# EUROPEAN PATENT SPECIFICATION

(11) **EP 1 330 546 B1**
(45) Date of publication and mention of the grant of the patent: **11.01.2006**
(21) Application number: 01978771.2
(22) Date of filing: 01.11.2001
(51) Int. Cl.: C12Q 1/68

(54) **METHOD FOR THE AMPLIFICATION AND OPTIONAL CHARACTERISATION OF NUCLEIC ACIDS**
METHODE ZUR AMPLIFIZIERUNG UND MÖGLICHER CHARAKTERISIERUNG VON NUKLEINSÄUREN
PROCEDE D'AMPLIFICATION ET DE CARACTERISATION SUPPLEMENTAIRE D'ACIDES NUCLEIQUES

(30) Priority: 03.11.2000 IE 20000887
(43) Date of publication of application: 30.07.2003
(73) Proprietor: University College Cork-National University of Ireland, Cork, Cork (IE)
(72) Inventor: McCARTHY, Thomas, Valentine, Cork (IE); COLLINS, Ruairi, Kildorrey, County Cork (IE)
(74) Representative: Ryan, Anne Mary
(86) International application number: PCT/IE2001/000139
(87) International publication number: WO 2002/036821

(56) References cited:
- EP-A- 0 497 272
- WO-A-97/12061
- WO-A-98/39485

## Description

### Technical Field

This invention relates to a new method for amplifying and characterising nucleic acids.

### Background Art

Extension of a nucleic acid primer on a target nucleic acid template is a highly important process with multiple applications including nucleic acid detection, diagnosis, and quantitation. In particular, extension of a primer on a template is i) direct evidence that the primer has annealed to the template, ii) confirms the presence of a sequence complementary to the primer on that template, and therefore iii) confirms the presence of that template or target nucleic acid. Using closely related primers that differ by as little as a single base, it is routinely possible to distinguish between nucleic acids that differ in sequence by a single base. The key limitation in using extension of primer on a template nucleic acid as a method for detection, diagnosis, and quantitation of nucleic acids is that in a typical extension reaction, the primer anneals (hybridises) to the template but is only extended once. Thus, the amount of template in a sample determines the amount of primer which is extended. For the majority of applications in the nucleic acid detection, diagnosis, and quantitation fields, the amount of template is often too small to permit direct detection of the extended primer if that extension reaction is not repeated or cycled in some manner.

Amplification processes are necessary to overcome this key limitation. A variety of these processes have been described previously and they essentially involve either: a) cyclic dissociation conditions where the extended primer is dissociated from the template using heat, thereby allowing annealing and subsequent extension of new primer, b) repeated generation of a primer in DNA through the use of a restriction enzyme to nick an unmodified strand of a hemi-modified DNA recognition site and the ability of a 5'-3' exonuclease deficient DNA polymerase to extend the 3' DNA terminus at the nick and displace the downstream DNA strand, c) where the template nucleic acid is circularised so that primer extension progresses continuously and/or d) where the copies of the template are produced by a transcription method which serve as templates in subsequent primer extension reactions.

Amplification of a nucleic acid (generating multiple copies) to the level where they can be detected and manipulated has a very high utility. Such amplification processes, generating sufficient amounts of a specific target nucleic acid are generally the first key step required in the characterisation of the nucleic acid. Direct amplification of a specific nucleic acid sequence from a sample permits diagnosis of the presence or absence of said nucleic acid in that sample and thus has a very high utility in DNA / gene diagnostics. Direct amplification of a nucleic acid from a sample and subsequent characterisation can be performed for a variety of purposes including diagnosis of the presence or absence of DNA variations such as mutations and polymorphisms in a specific nucleic acid. Amplification processes can in many cases be designed to permit both amplification and full or partial characterisation of the amplified target.

The current known methods for amplifying and characterising nucleic acids each have different limitations especially with respect to the process required for primer dissociation, primer generation, specificity, versatility, generation of single stranded DNA and facilitation of multiplexing and high throughput genotyping especially genotyping of single nucleotide polymorphisms (SNP) as described below.

### Primer extension

Primer extension *per se* on a template is very well documented in the literature. This can be achieved by annealing a primer to its complementary sequence on a template or target nucleic acid followed by incubation with a DNA polymerase in the presence of DNA precursors, typically dATP, dGTP, dCTP and dTTP, which results in extension of the primer from its 3'-OH (hydroxy) terminus in a 5' to 3' direction (with respect to the primer) on the template strand and produces a newly synthesised DNA strand that is complementary to the original template. Primer extension can only occur if the primer has a free 3'-OH terminus. Amplification of this complementary strand from the template by primer extension methods requires that a) primer extension occurs more than once per annealed primer molecule, b) new primer is repeatedly allowed to anneal to the template and be extended and / or c) that the complementary strand serves as a template for subsequent extension of a second primer.

### Polymerase chain reaction (PCR)

Amplification of a nucleic acid template can be achieved by the polymerase chain reaction (PCR) (Saiki, R.K. et al., Science. 239:487-491 (1988).

Typically, amplification of a target segment of a nucleic acid template by PCR is carried out using appropriate synthetic oligonucleotide primers in the PCR along with a thermostable DNA polymerase and DNA precursors. Multiple cycles of denaturation, annealing and primer extension results in the exponential amplification of the target segment. Thus detection of the product indicates the presence of a certain sequence at a specific locus. The length of the amplified product is determined by the combined length of the primers and the distance between their 3' termini on the template and so on.

PCR has an absolute requirement for a thermostable DNA polymerase. It also involves a thermal cycling process, therefore automation of this technique requires specialised equipment. A typical reaction normally requires two primers to initiate the reaction, therefore this adds additional complexity to the reaction, especially when one wants to consider multiplexing several different amplification reactions (i.e. in one tube). This leads to an increased number of primers present in the reaction and thereby increases the possibility of false and nonspecific amplification. In addition, there is also an increased complexity in designing and optimising the PCR reaction since the annealing temperature of the reaction has to suit both primers used in the reaction. This becomes an additional disadvantage when multiplexing different amplification reactions since a single annealing temperature must suit all amplification reactions being multiplexed.

Also, there is a potential for amplicon contamination since multiple copies of the template nucleic acid are produced during the reaction to act as subsequent templates.

### Transcription-based amplification methods

Transcription-based amplification (Kwoh, D.Y. *et al*., (1989) *Proc. Natl. Acad. Sci. USA, 86* 1173-1177; Guatelli, J.C. *et al.,* (1990) *Proc. Natl. Acad. Sci. USA, 87* 1874-1878; Compton, J. (1991) *Nature 350* 91-92.) is an amplification method that relies on primer extension of primers on a target nucleic acid so that a RNA polymerase promoter is created upstream of the target region to be amplified. Essentially a pair of primers that flank the target sequence are incubated with a template nucleic acid. One of the primers has an RNA polymerase promoter sequence upstream (5') of a sequence that is complementary and anneals to the template. The other primer is complementary to a segment of the complementary template strand. The primer with the RNA promoter sequence is extended on the template strand using a nucleic acid polymerase such as reverse transcriptase and DNA precursors. After thermal denaturation of the hybrid nucleic acid (the template strand and the newly synthesised complementary strand) or enzymatic degradation of the template strand, the second primer anneals to, and is extended on the newly synthesised complementary strand. This results in a product that is double stranded and has a RNA polymerase promoter attached to the target sequence. Incubation of this product with RNA polymerase and RNA precursors results in production of numerous RNA transcripts of that target sequence. Each RNA transcript serves in turn as a template for the production of a complementary DNA strand and this process continues in a self sustained cyclic fashion under isothermal conditions until components in the reaction become limiting or inactivated. This results in a large amplification of the target nucleic acid sequence.

One main disadvantage is that these techniques have an absolute dependence on RNA which is inherently more unstable than DNA and is more susceptible to degradation. Hence the reaction is ultra sensitive to contaminating ribonucleases. Typically the method requires RNA as template and is not suited or optimal with a DNA template.

Also the method requires at least two primers to initiate the reaction, where one of the primers must be specially designed to incorporate a transcription initiation site for a RNA polymerase.

Furthermore, there is potential for amplicon contamination since multiple copies of the template nucleic acid are produced during the reaction to act as subsequent templates.

These techniques are not suited to mutation or polymorphism detection without the addition of further steps.

### Strand Displacement Amplification (SDA)

SDA is an amplification method based upon the ability of a restriction enzyme to nick the unmodified strand of a hemi-modified double stranded DNA at a specific recognition site and the ability of a 5'-3' exonuclease deficient DNA polymerase to extend the 3' terminus at the resulting nick and in doing so, displacing the downstream DNA strand (Walker, G.T. et al., PNAS 89:392-396 (1992)). Exponential target DNA amplification is achieved by coupling reactions on the template in which strands displaced from the reaction on the template strand serve as target for the reaction on the complementary strand and *vice versa.* Essentially heat denaturation of a DNA sample generates two single stranded DNA fragments (T1 and T2). Present in excess are two DNA amplification primers (P1 and P2). The 3' end of P1 binds to the 3' end of T1, forming a duplex with 5' overhangs. Likewise, P2 binds to T2. The 5' overhangs of P1 and P2 contain a recognition sequence for a restriction enzyme such as HincII. An 5'-3' exonuclease deficient form of *E. coli* DNA polymerase extends the 3' ends of the duplexes using the DNA precursors dGTP, dCTP, dTTP and the modified precursor deoxyadenosine 5'-[alpha thio] triphosphate, which produces a hemiphosphorothioate recognition sites on P1T1 and P2T2. HincII nicks the unprotected primer strands of the hemiphosphorothioate recognition sites, leaving intact the modified complementary strands. The DNA polymerase extends the 3' end at the nick on P1T1 and displaces the downstream strand that is functionally equivalent to T2. Likewise, extension at the nick on P2T2 results in displacement of a downstream strand that is functionally equivalent to T1. Nicking and polymerisation/displacement steps cycle continuously on P1T1 and P2T2 because extension at the nick regenerates a nickable HincII recognition site. Target amplification is exponential because strands displaced from P1T1 serve as targets for P2 and strands displaced from P2T2 serves as targets for P1.

A major disadvantage of SDA is that one must use specially designed primers which incorporate a site for a specific restriction enzyme. Typically one requires two or more sequence specific primers *per* amplicon to carry out the reaction. In addition, the amplified fragments produced in this reaction using a single primer do not readily have a defined 3' terminus. This is also a major disadvantage of SDA since a defined 3' terminus on a displaced fragment allows one to prime a subsequent reaction with this same fragment.

WO 97/03210 discloses a method for rapidly detecting the presence or absence of a particular nucleic acid sequence at a candidate locus in a target nucleic acid sample comprising the steps of: 1) introducing a modified base which is a substrate for a DNA glycosylase into said candidate locus at one or more preselected positions; ii) excising the modified base by means of said DNA glycosylase so as to generate an abasic site; iii) cleaving phosphate linkages at abasic sites generated in step ii); and iv) analysing the cleavage products of step iii) so as to identify in said target nucleic acid sequence the presence or absence of said particular nucleic acid sequence at said candidate locus. The method has particular application for detecting specific mutations in a DNA sample, including the detection of multiple known mutations in DNA.

WO 99/54501 discloses a method for characterising nucleic acid molecules comprising the steps of i) introducing, a modified base, for example uracil, which is a substrate for a DNA glycosylase into a DNA molecule; ii) excising the modified base by means of said DNA glycosylase so as to generate an abasic site; iii) cleaving the DNA at the abasic site so as to generate an upstream DNA fragment that can be extended; and iv) incubating the extendible upstream fragment in the presence of an enzyme, for example, a polymerase or a ligase, allowing for extension thereof and a template nucleic acid and analysing the resultant fragment(s). However, in the case of the method described in WO 99/54501 for the cleavage of the DNA at the site where the base was excised is critical.

In the case of the method as exemplifed in WO 99/54501 the reaction mixture bearing the amplified target nucleic acid was treated with exonuclease I to digest the primers not extended in the amplification step and shrimp alkaline phosphatase to digest dNTPs not incorporated during the amplification step. Accordingly, no further amplification of the template nucleic acid occurred and the method was limited to a single cycle.

Consequently, it is important to develop an improved method for amplification and characterisation of nucleic acids that is more versatile, more specific, offers higher throughput, facilitates multiplexing of reaction and permits generation of single stranded DNA.

Accordingly, the invention provides a method for the amplification of a template nucleic acid, which comprises simultaneously carrying out the steps of:
i) reacting a nucleic acid primer with said template nucleic acid, normal DNA precursor nucleotides, at least one modified DNA precursor nucleotide and a DNA polymerase so as to obtain an extended nucleic acid primer, said nucleic acid primer remaining bound to said template;
ii) cleaving the modified base-containing extended nucleic acid primer so as to generate a free 3'-OH terminus that is extendible by said DNA polymerase; and
iii) repeating steps i) and ii) on DNA fragments thereby generated.

In one embodiment, the modified base-containing extended nucleic acid primer is cleaved by a 3'-endonuclease.

In this embodiment, preferably, the 3'-endonuclease is Endonuclease V from *E. coli* or homologues thereof to be found in other organisms.

In an alternative embodiment, the method comprises the steps of:
i) reacting a nucleic acid primer with said template nucleic acid, normal DNA precursor nucleotides, at least one modified DNA precursor nucleotide which is a substrate for a DNA glycosylase, and a DNA polymerase so as to obtain an extended nucleic acid primer, said nucleic acid primer remaining bound to said template;
ii) excising the modified base of the modified DNA precursor nucleotide from the extended nucleic acid primer by means of a DNA glycosylase so as to generate an abasic site;
iii) cleaving the extended nucleic acid primer at the abasic site so as to generate a free 3'-OH terminus that is extendible by said DNA polymerase; and
iv) repeating steps i)-iii) on DNA fragments thereby generated.

The method according to the invention has many specific advantages as set out below. However, more generally the method according to the invention has significant advantages over existing technologies in that it is more versatile and more flexible with respect to providing a single high throughput process that can be easily adapted to multiple different formats in the fields of DNA detection, quantitation and characterisation.

The invention will be described principally hereinafter with reference to the embodiment involving the use of a DNA glycosylase. We have coined the term glycosylase mediated amplification (GMA) for this method according to the invention. However, all embodiments of the invention are referred to collectively herein under the acronym GMA.

In the method according to the invention the modified DNA precursor nucleotide can thus be a substrate for a DNA glycosylase or recognised by a 3'-endonuclease as described herein.

Typically, the nucleic acid template strand can be any strand from a natural or artificially synthesised nucleic acid.

Preferably, the template nucleic acid is DNA.

The method according to the invention is primed/initiated by the nucleic acid primer. For convenience, the primer responsible for initiating the reaction is referred to herein as the initiating primer (IP).

The IP can be any nucleic acid with a free 3'OH terminus that can be extended by a DNA polymerase. The IP may be artificially synthesised for example a synthetic oligonucleotide, or derived directly or indirectly from a naturally occurring nucleic acid.

In one embodiment, the nucleic acid primer is a DNA primer.

The normal DNA precursor nucleotides are the deoxyribonucleotide triphosphates dATP, dCTP, dGTP and dTTP. In certain limited circumstances, dideoxynucleotide triphosphates may also be used and included in the reaction. Therefore, possible normal precursors also include ddATP, ddCTP, ddGTP and ddTTP.

Preferably, the DNA precursor nucleotides are selected from dATP, dCTP, dGTP and dTTP.

Any one of several nucleic acid polymerases may be used in GMA. When a DNA template is used a DNA polymerase is used. When an RNA template is used a DNA polymerase which can utilise a RNA template is required, typically such an enzyme is reverse transcriptase. Typically, two classes of DNA polymerases are used depending on whether displacement or digestion of the nucleic acid downstream of the extending primer is required. When strand displacement is required, thereby generating displaced fragments, a DNA polymerase that does not have a 5'-3' exonuclease activity is used. In contrast, when a DNA polymerase with 5'-3' exonuclease activity is used, the downstream DNA is degraded in each cycle of the GMA reaction. This also leads to generation of a detectable product as discussed further below.

There are several known modified precursor nucleotides which, when incorporated into DNA, become a substrate for a DNA-glycosylase and/or are recognised by a 3'-endonuclease, as appropriate. In the latter case, the modified precursor nucleotide directs cleavage by a 3'-endonuclease enzyme to a phosphodiester bond 3' to the site of incorporation thereof.

In each case cleavage is dependent on the presence of the modified base in the DNA and it is this that dictates the cleavage of the DNA and dictates the location of the cleavage, in either of two ways, namely, 1) excision of the modified base by the glycosylase and cleavage of the subsequent abasic site or 2) cleavage of the extended nucleic acid primer at the second phosphodiester bond on the 3' side of the site of incorporation of the modified base by a 3'-endonuclease enzyme. In one embodiment, the modified nucleic acid precursor is dUTP.

The modified precursor nucleotide dUTP is a base sugar phosphate comprising the base uracil and a sugar phosphate moiety. Primer extension on template using the precursor nucleotides dATP, dCTP, dGTP, and dUTP in place of dTTP, results in newly synthesised DNA complementary to the template where thymine is replaced completely by uracil.

However, it will be appreciated by those skilled in the art that other modified nucleic acid precursors can also be used, such as dITP and 8-OH dGTP.

The modified precursor nucleotide dITP is a base sugar phosphate comprising the base hypoxanthine and a sugar phosphate moiety. The modified precursor nucleotide 8-OH dGTP is a base sugar phosphate comprising the base 8-OH guanine and a sugar phosphate moiety.

The glycosylase substrate precursors dUTP, dITP and 8-OH dGTP when incorporated into DNA generate the glycosylase substrate bases uracil, hypoxanthine and 8-OH guanine, respectively.

In one embodiment, the DNA glycosylase is uracil DNA-glycosylase (UDG).

Uracil in DNA is recognised specifically by UDG and released from DNA. UDG also recognises other uracil related bases when present in DNA.

Many DNA-Glycosylases have been described. These enzymes cleave the N-glycosidic bond connecting the glycosylase substrate base to the DNA backbone. This releases the base from the DNA and generates an abasic site.

Other suitable DNA glycosylases include alkylpurine DNA-glycosylases (ADG) or formamidopyrimidine DNA-glycosylase (FPG) DNA-glycosylase.

Hypoxanthine is recognised specifically by alkylpurine DNA-glycosylases (ADG) and released from DNA. This enzyme also recognises and releases N3 methyladenine, N3 methylguanine, O² methylcytosine and O² methylthymine when present in DNA. 8-OH guanine is recognised specifically by FPG DNA-glycosylases and released from DNA. This enzyme also recognises and releases ring opened purines when present in DNA.

Several agents are known which cleave the phosphodiester bonds in nucleic acids at abasic sites. Cleavage of the bond can be 5' of the abasic site or 3' of the site. 5' cleavage can occur proximal or distal to the phosphate moiety and generate an upstream fragment which has a 3' terminus with a free 3'OH group or 3'-phosphate group respectively. 3'-OH termini are extendible by DNA polymerases on a template whereas 3'-phosphate termini are not extendible. Such 3'-phosphate termini can generally be rendered extendible by treatment with a phosphatase enzyme such as T4 polynucleotide kinase which has a 3' phosphatase activity. Agents which cleave 5' to the phosphate moiety and generate a 3' terminus with a free 3'OH are the enzymes with AP endonuclease activity, such as AP endonuclease IV from *E. coli*. Agents which cleave 3' to the phosphate moiety and generate a 3' terminus with a 3'-P group are alkali, heat and certain DNA repair enzymes such as FPG and basic proteins and peptides. Agents which cleave 3' of the abasic site include heat, and DNA repair enzymes with AP lyase activity such as endonuclease III from *E. coli.* Such 3'-deoxyribophosphate (dRp) termini can generally be rendered extendible by treatment with an AP endonuclease. FPG-DNA glycosylase cleaves both 5' and 3' of the abasic site.

Preferably, the extended nucleic acid is cleaved at the abasic site by means of an enzyme which cleaves at a nucleic acid abasic site.

Further, preferably, the enzyme is an AP endonuclease, especially AP endonuclease IV which cleaves 5'of the abasic site and generates a free 3' OH terminus.

Where the extended primer is cleaved by a 3'-endonuclease, cleavage of the extended primer by such an enzyme is dependent on the presence of a modified base in the extended primer and cleavage occurs at a phosphodiester bond on the 3' side (i.e. downstream) of the site of incorporation of the modified base. In contrast to the action of the glycosylase and AP site cleavage, cleavage of the extended primer by the 3'-endonuclease does not involve excision of the modified base and does not involve creation of an abasic site. Cleavage by the 3'-endonuclease is dependent on the presence of a modified base in the extended primer and recognition of said modified base by the enzyme. Cleavage usually occurs at the second phosphodiester bond on the 3' side of the modified base/nucleotide. This cleavage event results in the generation of a nick in the DNA strand with a 3'-OH and a 5'-phosphoryl group being generated. The DNA polymerase present in the reaction can then extend from the free 3'OH group. As mentioned above, the 3'endonuclease may be Endonuclease V from *Escherichia coli.* Endonuclease V recognises several modified bases in DNA including uracil, hypoxanthine (inosine) and urea residues. In addition to cleavage of DNA with modified bases, endonuclease V can also cleave DNA containing abasic sites. Therefore in certain circumstances, endonuclease V could be used to cleave the extended primer in combination with the action of a DNA glycosylase which generates an abasic site in the DNA.

In one embodiment, the modified precursor nucleotide partially replaces one of the normal precursor nucleotides.

For example, primer extension on a template using the precursor nucleotides dATP, dCTP, dGTP and dTTP in addition to the modified precursor nucleotide dUTP results in newly synthesised DNA complementary to the template where thymine is replaced randomly by uracil. The uracil is incorporated in the newly synthesised DNA strand at positions complementary to adenine residues in the template DNA strand during the DNA synthesis process. Thus, the downstream displaced fragments are delimited by the position of the random incorporation of the dUMP in the newly synthesised DNA. This results in the generation of displaced fragments of multiple sizes according to the permutations of dUTP *versus* dTTP incorporation in the complementary strand opposite A residues in the template nucleic acid strand.

A similar approach substituting dITP for dGTP or 8-OH dGTP for dGTP leads to replacement of all or part of dGTP in the newly synthesised DNA complementary to the template by hypoxanthine or 8-OH guanine, respectively at positions complementary to cytosine residues in the template DNA strand. Replacement of all or part of one or more of the regular DNA precursors with one or more dideoxy terminator nucleotides (a nucleotide that prohibits further extension of a primer on a template once incorporated) can be used to terminate primer extension by a DNA polymerase when desired. Replacement of part of one or more of the regular DNA precursors with one or more dideoxy terminator nucleotides terminates primer extension at multiple different positions on the template strand and generates terminated primers of multiple different lengths. Replacement of all of one or more of the regular DNA precursors with one or more dideoxy terminator nucleotides terminates primer extension at specific positions on the template strand and generates terminated primers of specific lengths. More than one modified precursor nucleotide may be used in the GMA with one or more DNA-glycosylases. Two DNA glycosylases may be used whereby one releases a modified base from the primer while the other releases the modified base once incorporated into newly synthesised DNA.

Steps i) and ii) or i)-iii), as appropriate of the method according to the invention can continue in a cyclical manner until one of the reagents becomes limiting.

The method according to the invention can be carried out under isothermal conditions.

Accordingly, when the method is isothermal, no thermocycling is required.

The method according to the invention is the only isothermal amplification reaction capable of amplifying multiple, newly synthesised and discrete DNA segments in a primer extension reaction using a single primer.

It will be appreciated that the method according to the invention can result in the accumulation of displaced single stranded downstream fragments of nucleic acid specified by the locations of modified bases in a complementary nucleic acid strand:

Thus, the method according to the invention provides a means of generating multiple copies of discrete single stranded primers downstream of an initiating primer. This offers exceptional specificity for detection purposes, as the discrete downstream primers can only be generated if the target template nucleic acid is present. Thus for DNA diagnosis, GMA is a significant improvement over any previously described amplification method with respect to specificity.

Amplification of multiple DNA segments from a single template sample is highly desirable and is currently a limitation for amplification technologies. This limitation largely arises from the fact that existing technologies use exponential amplification and/or are more cumbersome and generate double stranded product or large size single stranded product.

The method according to the invention offers significant advantages over existing methods for multiplex amplification of DNA segments.

The method according to the invention can be used for generating multiple copies of discrete single stranded primers downstream of an initiating nucleic acid primer.

The displaced downstream fragments can be extended in a secondary reaction.

Furthermore, the displaced downstream fragments can be extended on a secondary template nucleic acid.

Also multiple secondary templates can be immobilised on a DNA chip.

These aspects of the invention will be described further below.

The method according to the invention can be used in detection diagnostics. Thus, for example the method can be used in the detection of pathogens.

The method according to the invention can also be used in the detection of the presence or absence of mutations and in the detection of polymorphisms.

It will be appreciated that the method according to the invention can be used in the quantification of the level of a nucleic acid in a sample.

The GMA method according to the invention can be measured both qualitatively and quantitatively by several means. Therefore, the characteristics and quantity of an IP and / or its complementary annealing site on a template nucleic acid can be assessed by the ability of the IP to prime a GMA reaction on that template. The resolution achieved according to the invention, if desired, can be as high as determining single base differences between IPs and /or the template or target nucleic acid and this is based on the successful priming of a GMA reaction. Since the IP may be derived directly or indirectly from a naturally occurring nucleic acid, and the GMA permits qualitative and quantitative characterisation of the IP, the GMA can therefore be used to qualitatively and quantitatively characterise nucleic acids. This has high utility in the fields of detection, diagnosis, and quantitation of nucleic acids. This includes for example, detection and quantitation of pathogenic micro-organisms like certain bacteria and viruses, detection of variants therein, detection of human disease causing mutations, detection of single nucleotide polymorphisms and quantification of the amount/titre of specific mRNA species in tissue samples.

The method according to the invention has significant advantages over existing technologies in the area of quantitation. As the kinetics of the GMA are linear, the GMA reaction is easier to detect and measure/quantify than existing amplification technologies with exponential kinetics.

The method according to the invention also has significant advantages over existing technologies in the area of contamination control. The reason for this is that unlike existing technologies, GMA in its basic format does not synthesise new templates for subsequent use by initiating primers and the kinetics of the process are linear.

The method according to the invention can also be used in signal amplification from any nucleic acid that can function as a primer or template.

The method according to the invention has significant advantages over existing technologies in that it permits signal amplification from an initiating primer using a single linear template. The GMA does not incorporate the initiating primer into the amplified displaced downstream fragments. This offers the advantage that the displaced downstream fragments do not have a 5' tail that is always the initiating primer. Furthermore, this means that the displaced downstream fragments can be extended in a secondary reaction to produce complementary displaced downstream fragments devoid of sequences complementary to the initiating primer.

The method according to the invention is unique in that it can be carried out in a single reaction vessel whereby extension of an IP on a template generates and amplifies new primers that are distinct from the IP and which can subsequently serve as IPs on the same template from which it was derived or on a different template.

### Brief Description of Drawings

Fig. 1 is a flow diagram of one embodiment of the method according to the invention as described *inter alia* in Example 1; and
Fig. 2 is a flow diagram of another embodiment of the method according to the invention as described in Example 4.

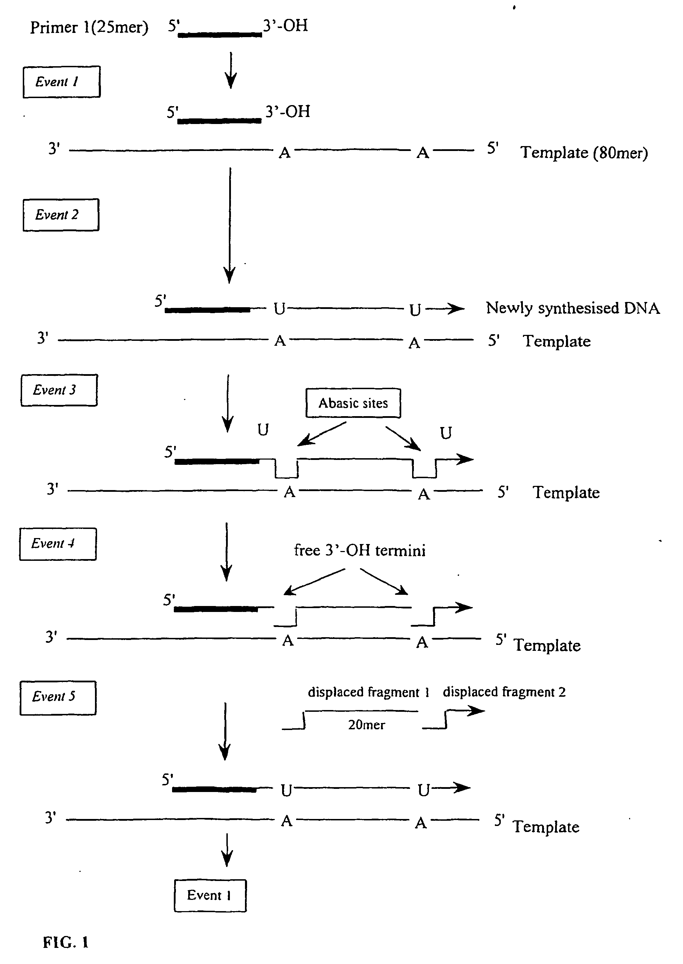

One embodiment of the invention is illustrated with reference to Fig. 1. as follows:
- Event 1: The primer binds to a complementary sequence on the template;
- Event 2: Once bound the free 3'OH terminus of the primer is extended by the DNA polymerase. This happens through the polymerisation of the precursor nucleotides onto the 3'OH terminus of the primer. The precursor nucleotides (dATP, dCTP, dGTP and / or dTTP) are incorporated into the extending primer in addition to the modified precursor nucleotide according to the sequence of the template. The modified precursor nucleotide usually replaces either fully or partially one of the normal precursor nucleotides. The newly synthesised DNA strand is complementary to the initial template and is referred to as the complementary template strand herein;
- Event 3: Once the modified precursor nucleotide is incorporated into the newly synthesised DNA, that DNA then contains a modified base which is a substrate for the specific DNA glycosylase. Consequently every time a modified base appears in the newly synthesised DNA, it is released from the DNA by cleavage of the N-glycosylase bond that joins that base to the deoxyribose moiety in the DNA. This results in the production of an abasic site which is essentially a deoxyribose moiety joined to the flanking DNA by a phosphodiester bond on the proximal and distal side (i.e. 5' and 3' of the deoxyribose moiety with the 5' bond being the closest to the original primer); and
- Event 4: The abasic site is for example a substrate for AP endonuclease (APE) enzyme. Consequently every time an abasic site appears, it is cleaved by APE. This enzyme cleaves the phosphodiester bond 5' of the deoxyribose moiety generating a free 3'OH terminus on the upstream DNA segment and a deoxyribose moiety attached to the 5' end of the downstream segment.
- Event 5: The DNA polymerase present in the reaction synthesises new DNA from this newly generated 3'OH terminus of the upstream fragment every time it is created, and in doing so, displaces the DNA downstream of the polymerisation as a single strand. This results in the incorporation of new precursors nucleotides, including modified precursor nucleotides, into the newly synthesised complementary template strand and the appearance of a new modified base at each position in the newly synthesised strand opposite its complementary base in the template nucleic acid.

Thus, the reaction steps i) to iii) according to this embodiment cycle continuously until one of the reagents becomes limiting.

Each free 3'OH terminus created in a cycle of the reaction is extended once in each subsequent cycle of the reaction with concomitant displacement of the downstream DNA segments. As the reaction is continuous the net result is the repeated synthesis of new DNA from each 3'OH terminus created and accumulation of the displaced downstream DNA as discrete single stranded fragments of discrete sizes, referred to herein as displaced downstream fragments, or displaced fragments, delimited by the locations of modified bases in the complementary strand and/or the 3' terminus due to termination of DNA synthesis by the polymerase.

Primers that are extended and cleaved can be immediately re-extended by the polymerase by incorporation of nucleotides including modified precursor nucleotides. Since the normal precursor nucleotides, modified precursor nucleotides, polymerase, glycosylase and cleavage agents are all simultaneously present in the same reaction, a continuous cycle of extension and cleavage results in the amplification of multiple copies of displaced downstream fragments.

When the modified precursor nucleotide is dUTP, then the modified base is uracil and the specific DNA glycosylase when such is used is Uracil DNA glycosylase. Hence the displaced downstream fragments are delimited or defined by the locations of uracil in the complementary strand and therefore by the location of adenine bases in the template nucleic acid, since uracil forms a normal Watson-Crick base pair with adenine.

Therefore, briefly, the primer binds to the template and is extended by the DNA polymerase. DeoxyATP, dCTP, dGTP, and dUTP, are incorporated into the extending primer. Uracil DNA glycosylase then excises the uracil bases in the newly synthesised strand and the resulting abasic sites are cleaved by the AP endonuclease enzyme.

Alternatively, the 3'-endonuclease recognises uracil in the newly synthesised strand and cleaves the strand at the second phosphodiester 3' of the uracil moiety.

The DNA polymerase then begins to synthesise new DNA from the newly generated 3'OH termini every time they are created and displaces the 3' or downstream DNA as the polymerisation proceeds. This again results in the incorporation of more uracil into the newly synthesised DNA which is subsequently excised and/or recognised', the DNA cleaved and polymerisation initiated from the new 3'OH terminus.

The GMA may be carried out at mesophilic or thermophilic temperatures. A DNA polymerase such as the *E. coli* DNA polymerase Klenow fragment exo may be used at mesophilic temperatures (usually between 25°C and 42°C and typically at 37°C) while at thermophilic temperatures (typically between 50°C and 80°C, although it can be higher), thermostable DNA polymerases such as a strand displacing DNA polymerase from *Thermus aquaticus* (Stoffel fragment) may be used. Both types of polymerase may be added jointly or sequentially to a reaction. When high processivity is required, so that a primer is extended to a considerable length before the polymerase disassociates from the DNA, a highly processive polymerase can be used. By contrast, when low processivity is required, so that a primer is extended to a short length before the polymerase disassociates from the DNA, a lowly processive polymerase can be used. When an RNA template is used, a reverse transcriptase with strand displacement activity may be used for the GMA reaction.

In the simplest case, an artificial or synthetic primer is supplied as the IP to prime the GMA reaction on a given template or target nucleic acid. The IP is chosen so that it hybridises to a specific target sequence in the template. Following hybridisation of the IP, the GMA initiates and the extended IP is repeatedly extended in the cyclic reaction resulting in amplification of the displaced downstream DNA fragments. These displaced fragments can be qualitatively and quantitatively characterised by multiple different approaches according to published procedures.

Direct detection of displaced fragments can be achieved by a variety of means, for example they may be suitably labelled.

Labelling of the displaced fragments can be performed by a variety of means including addition of a radioactive, fluorescent, or detectable ligand to the fragments during or post synthesis. The use of a labelled precursor nucleotide in any of the extension reactions facilitates detection of these fragments. Direct DNA staining methods such as silver or ethidium bromide staining facilitates their detection after size separation based on electrophoretic mobility. Hybridisation of complementary or test nucleic acids to these fragments may be used to identify them and such complementary or test nucleic acids may be immobilised and hybridised directly to the displaced fragments. In this context, DNA macroarrays, DNA microarrays and DNA chips are very suitable. Alternatively, the displaced fragments may serve as a bridging hybridisation molecule so that one test nucleic acid, which may be immobilised, is hybridised to part of a displaced fragment and a second test or reporter nucleic acid hybridises to the remainder of the displaced fragment. Again, DNA macroarrays and DNA microarrays are also very suitable in this context.

The complementary or test nucleic acids may suitably be labelled by any of a variety of direct or indirect labelling approaches such as reporter-quencher fluorescent dye methods. Since the displaced fragments are single stranded, hybridisation to complementary molecules will create double stranded nucleic acids which may be detected using double stranded specific probes such as SYBR green. This may be achieved in the GMA reaction according to the invention by including DNA complementary to the displaced fragments in addition to SYBR green reagent which specifically binds double stranded DNA.

The sequence of the displaced fragments and, in particular, the 3'end thereof can be determined by their ability to function as initiating primers in a subsequent or the same GMA reaction. Essentially, such determination is based on the ability of these fragments and particularly their 3'end to hybridise to a selected complementary sequence on a template under selected conditions and to function as an IP in a secondary GMA reaction. It will be appreciated that multiple possibilities exist for the selection of complementary sequences on a template and template molecules themselves. Nonetheless, the ability of a displaced fragment to function as an IP in its own right in a GMA reaction is a measure of its hybridisation to, or lack of hybridisation to, a selected target sequence and thus the determination of the nature of the sequence of part or all of the displaced fragment is on this basis.

Detection of the displaced fragment is highly advantageous from a specificity perspective since the generation thereof is dependent on a) the successful hybridisation of the IP to the target template and b) priming of a GMA reaction on the correct template. Thus, detection of the anticipated displaced fragment is evidence that the IP has hybridised to the correct region on the correct template.

The identity or sequence of the displaced fragment can be determined using a variety of approaches including hybridisation, mass measurement, and the ability thereof to be ligated directly to a nucleic acid or its ability to act as the complementary strand necessary for ligation of one or more nucleic acid molecules. For example, it can be detected and characterised by assessing the ability thereof to serve as a bridging hybridisation molecule for ligation of the 5' and 3' end of a linear test DNA to form a circle. The hybridised displaced fragment, or an additional primer, can then serve as an IP for a GMA reaction on the new circular template resulting in amplification of the DNA by a rolling circle replication (RCR) mechanism.

It should also be noted that, in addition to acting as an IP, a displaced downstream fragment can also act as a template in a subsequent GMA reaction.

There are many methods by which an IP can be generated. In all cases the IP supplied or generated must have a free 3'OH terminus so that it can prime the subsequent DNA polymerisation step.

Artificial synthesis of an IP allows for numerous possibilities with respect to synthesis, design and modification of the IP. Many different modifications of artificially synthesised primers have been previously described. These include modifications of the base, sugar and phosphodiester bond and including those whereby glycosylase substrate bases such as uracil, hypoxanthine and 8-hydroxy guanine are incorporated into the primer.

Typically a standard or modified IP is synthesised to specifically match all or part of its complementary sequence on the template nucleic acid. It is well established that complementarity between the bases at the 3' end of a potentially extendable primer and the template is one of the key parameters that determines whether the IP will be extended on that template. An IP that is fully complementary to a section of the template can be extended by DNA polymerase, whereas an IP that is fully complementary to a section of the template except for the base at its 3' terminus is not extended under stringent conditions. Thus, it will be appreciated that extension of an IP on a template can be used to differentiate between closely related IPs that differ by as little as a single base. Similarly, it will be appreciated that extension of an IP on a template can be used to differentiate between closely related templates that differ by as little as a single base. This approach is of particular importance in the field of human genetics where it permits detection of mutations and polymorphisms such as single nucleotide polymorphisms (SNPs).

It is well established that the stringency of hybridisation conditions between IP and a template can be varied considerably. Low stringency conditions permit low specificity hybridisation between DNA molecules. Thus under low stringency conditions, DNA molecules that are partially complementary can hybridise to each other. Therefore, under such conditions a partially complementary IP could hybridise to a template. Under such conditions, a single IP can hybridise and be extended at one or more partially complementary sites on a template nucleic acid. When the stringency conditions are so low that priming occurs at multiple sites, the process is referred to as random priming (although the priming is not entirely random in that a significant match between the five most 3' bases of the IP and the template is usually required). As stringency is increased, hybridisation becomes increasingly specific and hybridisation conditions can readily be found that permit only hybridisation of a fully complementary primer, but excludes hybridisation of a partially complementary primer even if it only differs by a single base. During hybridisation, there are a number of parameters by which the stringency of hybridisation may be altered, such as temperature. As temperature is increased, the stringency of hybridisation increases. Consequently, in enzymatic processes that are dependent on hybridisation between DNA molecules, higher specificity can be achieved at higher temperatures. However, the enzymatic process at the higher temperature usually requires thermostable enzymes.

The IP may be generated following cleavage of an artificially synthesised primer or of a natural nucleic acid such that a new free 3'OH terminus is produced. The cleavage can be either single or double strand dependent, dependent on the presence of a modified base in a single strand or double strand context, sequence dependent, dependent on the presence of a mismatch, or dependent on the presence of a specific structure. Thus, a primer may be generated through glycosylase mediated cleavage of a probe bearing a modified base that is recognised by a specific glycosylase in a single strand or double strand context. An IP may also be generated by cleavage of a probe bearing a mismatched base that is recognised by a mismatch specific endonuclease or glycosylase in a double strand context, for example when the probe is annealed to the template nucleic acid. This can be achieved by designing the primer so that a base mismatch is created between the primer and the template at one or more locations in the hybridised segment and incubating with one or more of a variety of endonucleases or DNA glycosylases which have been shown previously to specifically cleave at mismatched bases in double stranded nucleic acids. These include T7 endonuclease I, MutY DNA glycosylase, thymine mismatch DNA glycosylase and endonuclease V.

An IP may also be generated through cleavage of a complex formed by the hybridisation of overlapping oligonucleotide probes using a structure-specific enzyme such as a cleavase.

A primer may be synthesised with a blocked 3' terminus so that extension of the primer is not possible unless the blocking group is released through cleavage of the primer. Such a primer is referred to as a 3'-blocked primer herein. A blocked 3' terminus of a primer can be achieved by several methods including 3' phosphorylation, incorporating a dideoxy nucleotide at the 3' terminus, synthesising the primer with a 3'amine or 3'thiol group, synthesising the primer with one or more inverted nucleotides at the 3' terminus, or cleaving an abasic site with a DNA lyase.

Therefore in one embodiment of the invention, a primer may be synthesised with a noncomplementary 3' terminus (i.e. not complementary to the template at its 3' terminus) so that extension of the primer is not possible unless the 3' terminus is released through cleavage of the primer.

A primer may be synthesised with a noncomplementary 5' terminus so that cleavage causes dissociation of the primer from template nucleic acid. The dissociated primer can then serve as an IP in a GMA reaction on a different template.

Cleavage of a primer so that a 3'-blocked primer is unblocked, and a noncomplementary 3' terminus or noncomplementary 5' terminus is released, may be made dependent on hybridisation of the primer to all or part of a template nucleic acid so that following the cleavage, an IP with a new free 3'OH terminus is created, permitting extension of the IP on the same or a distinct template. Cleavage of the hybridised primer in this instance so that it can extend on a template, requires that the primer is cleaved at one or more locations in the hybridised segment of that primer. This can be achieved by designing a primer containing the modified base hypoxanthine which is a substrate for 3-alkylpurine DNA glycosylase (e.g. AlkA).

In a further embodiment of the invention, using thermostable cleaving agents, it is possible to cleave a hybridised primer at a modified or mismatched base so that once the probe is cleaved, the two or more fragments become thermally unstable and fall off the target nucleic acid, thereby allowing another full-length primer to hybridise. This oscillating process amplifies the signal (increased generation of the cleaved primer). The cleaved product with a 3'OH terminus can then serve as an IP in a subsequent or coupled GMA reaction.

An IP may also be generated by cleavage of a primer at a modified base where such cleavage is dependent on extension of the primer on a template. This permits generation of an IP smaller than the original primer and which can be characterised in a variety of ways. For example, *E. coli* Uracil DNA Glycosylase will not release uracil from the ultimate or penultimate 3' position of a primer. However, if the primer is extended on a template, the uracil which was at the ultimate or penultimate 3' position of the primer is now further away from the 3' terminus of the newly extended nucleic acid and will therefore be released. Thus a primer with a free 3'OH terminus and one or two bases shorter than the original primer is generated and is therefore an IP for a subsequent GMA reaction.

An IP may be generated from a naturally occurring or amplified nucleic acid by full or partial enzymatic or chemical cleavage with DNA or RNA cleaving agents such as DNAses, RNAses, restriction endonucleases, DNA glycosylases following conversion of a normal DNA base to a glycosylase substrate base or incorporation of such a base during amplification, AP endonucleases following partial or full depurination or depyrimidination of DNA, enzymes that cleave RNA or DNA in RNA:DNA hybrids such as RNAseH and enzymes that cleave at DNA mismatches formed following denaturing and re-annealing of nucleic acid hybrid molecules such as RNA:DNA hybrids and DNA:DNA hybrids. Enzymatic or chemical cleavage of DNA or RNA may generate 3' termini that are not extendible by DNA polymerases. Such 3' termini can generally be rendered extendible by treatment with one or more enzymes such as AP endonuclease IV or T4 polynucleotide kinase which has a 3' phosphatase activity. It is well established that cleavage agents can be double strand, single strand or sequence specific. A double or single stranded nucleic acid may be cleaved to small double or single stranded fragments, respectively using one or more cleavage agents prior to GMA. This provides a means to restrict the size of the displaced fragments.

Cleavage or nicking of one strand of a duplex molecule provides a section of nucleic acid with a free 3'OH terminus that can function directly as an IP on the template to which it is hybridised or on a distinct template in a GMA reaction. Cleavage or nicking of one strand of a duplex molecule can be achieved using certain cleavage agents. The nucleic acid can be nicked non-specifically with a low amount of nuclease enzyme such as DNAse, leading to the generation of multiple different displaced downstream fragments from multiple locations on the nucleic acid template.

The nucleic acid can be nicked with more specific agents such as the restriction enzyme N.BstNB I which nicks DNA four bases downstream of the 3' side of the recognition sequence GAGTC. Alternatively, the nucleic acid can be nicked with a restriction enzyme, for example HincII or BsoBI, which nicks the unmodified strand of a hemi-modified double stranded DNA at a specific recognition site, thereby generating a free 3'OH terminus. RNA may be cleaved non-specifically with certain RNAses and more specifically with sequence or structure specific RNAses such as ribozymes. RNAseH cleaves RNA in a RNA:DNA hybrid. Thus RNA can be cleaved by RNAseH following synthesis of a cDNA on the RNA template by reverse transcriptase. The RNA can be cleaved specifically by RNAseH following annealing of oligonucleotide DNA molecules to one or more sequences on the RNA.

In particular, addition of oligo / poly dT provides a means to make the 3'polyA tail of mRNA species double stranded. Addition of RNAseH results in digestion of the polyA tail providing a unique 3' terminus for each mRNA species. This method provides a means whereby each mRNA species in a sample can potentially act as an IP in a GMA.

A double stranded nucleic acid may be cleaved to expose its free 3'OH terminus enabling the cleaved DNA to function as an IP in a GMA. For example, a double stranded DNA may be denatured by heat. Alternately, it may be treated with the T7 gene 6 exonuclease which hydrolyses duplex DNA in a stepwise non-processive reaction from the 5' termini. The enzyme is not active on single stranded DNA and thus stops when duplex regions are no longer present.

Importantly, specific cleavage of one strand of a duplex molecule at a mismatch generates a nucleic acid fragment with a 3'OH terminus that can function directly as an IP on that template to which it is hybridised or on a different template. This provides a means of identifying mutations and polymorphisms in nucleic acids.

In this respect, the present invention allows one to investigate the presence or absence of a mutation or polymorphism at a specific location in a nucleic acid (candidate locus) in the following way: annealing a 3'-blocked primer to the target nucleic acid such that a mismatch is generated at the candidate locus. The mismatch is typically located internally with respect to the primer, i.e. that the base creating the mismatch with the candidate locus base on the template is not the base residue of the primer's 5' terminal or 3' terminal nucleotide. Upon cleavage of the primer at the position of the mismatch with a mismatch specific glycosylase and abasic site cleavage agent, the 5' cleaved section of the primer has a 3'OH terminus and is therefore an IP which can now initiate a GMA reaction on that same template or an alternative template. Therefore, the resultant GMA is indicative of the presence or absence of the mutation dependent on whether a mismatch was formed or not by the binding of the primer to the template and *vice versa*.

Additionally, reannealed nucleic acid hybrid molecules can be treated with mismatch specific enzymes such as T7 endonuclease I, MutY DNA glycosylase, thymine mismatch DNA glycosylase or endonuclease V. Combinations of gene amplification by PCR methods followed by reannealing and cleavage of DNA at mismatches with a mismatch specific repair enzyme generates free 3'OH terminii that can serve as IPs in a subsequent GMA reaction on a distinct template following dissociation from its complementary strand.

Alternatively, extension from the 3'OH terminus generated at the mismatched site using a strand displacement DNA polymerase in a standard "once off" strand displacement reaction, or in a GMA reaction, produces a displaced fragment that can serve as an IP in a subsequent GMA reaction. Since the generation of the IP is dependent on the presence of a mismatch, priming of a GMA reaction is indicative of a mismatch. When a whole genome amplification method is used with this approach with multiple probes and/or multiple templates for detection of displaced fragments, many amplification products can be tested for the presence of mismatches. Using selected probes and/or selected templates, it is possible to locate the mismatches. Using an array of probes, this can be applied to a genome wide search or to a search of multiple amplified nucleic acids simultaneously.

The use of a degenerate IP in a GMA reaction provides a means for priming the GMA at multiple sites on the template. Degeneracy of the IP may be very high and consequently random priming of a template may be achieved. In such a case, an IP such as a random hexamer, or a longer primer with a random hexamer as its 3' sequence, is used typically with a template such as genomic DNA. More specific multiple priming may be achieve with an IP with a lower level of degeneracy.

The method according to the invention can be used as a novel signal amplification method provided by the GMA. The extended IP and the displaced fragments created in a GMA reaction have a free 3'OH terminus and can function as primers that can be extended. Using a strand displacement DNA polymerase, the displaced fragments produced during the GMA on a template are free to function as IPs in a secondary GMA reaction, if a suitable template is available. A template may be provided in the same reaction or in an uncoupled reaction as a means for signal amplification. The template provided for use in signal amplification is referred to as the signal amplification template or booster template herein. This is especially important when the amount of the original template is low, which is often the case when genomic DNA is used as the template or target nucleic acid in the initial GMA. The booster template is synthesised so that it has an intrinsic sequence complementary to any initiating primer of interest, termed 'IP binding site' herein. Typically, this sequence is complementary to the extended initiating primer or a displaced fragment produced during a GMA reaction. Typically the IP binding site is at the 3' end of the booster template (the booster template is read in a 3' to 5' direction by the polymerase that polymerises and extends the IP in a 5' to 3' direction). One or more bases are present upstream (5') of the IP binding site on the booster template that are complementary to the modified base used in the GMA reaction. Typically, this base is an adenine residue. This results in the incorporation of a modified base, typically a U, at this position into the extending IP during the GMA reaction. The sequence following this position (upstream), referred to herein as the booster sequence is devoid of bases that are complementary to the modified base used in the initial GMA. The booster sequence can vary in size and is typically longer than 18 nucleotides. Typically, the booster sequence has a 3' modification such as an inverted nucleotide to prevent spurious self-priming. A DNA synthesis block may also be included in the booster template in the region complementary to the IP to prevent spurious self-priming. When an initiating primer primes a GMA on the booster template, it generates the complement of the booster sequence referred to as complementary booster sequence herein. The net effect of this procedure is that the complementary booster sequence is copied/amplified to a high level, as the booster template is typically not limiting, and every IP generated can potentially serve to prime any non-primed booster template in the GMA reaction. The complementary booster sequence can also serve as a universal reporter for detection purposes.

To achieve a very high level of signal amplification, the booster sequence (i.e. BS#1) can be followed by one or more bases that are complementary to the modified base in the GMA reaction. This booster sequence is followed in turn by a second booster sequence (BS#2). BS#2 is typically identical to BS#1. When an IP primes a GMA, reaction on the booster template, it generates the complement of BS#1 and BS#2 referred to as complementary booster sequence #1(cBS#1) and complementary booster sequence #2 (cBS#2) herein. cBS#1 and cBS#2 are identical and function as IPs priming the booster template from BS#1. They also bind to BS#2 but are displaced in each cycle of the GMA reaction. The net effect of this is that cBS#2 can be amplified to a high level as the booster template is typically not limiting and every cBS#1 and cBS#2 can potentially serve as initiating primers for any non-primed booster template in the GMA reaction.

There are many possibilities for booster template design. If cBS#1 is generated in the primary GMA reaction, then a booster template with only the BS#1 and BS#2 sequences, separated by a base complementary to the modified base, is necessary since the cBS#1 serves as the initiating primer. A booster template can be designed with several different IP binding sites, i.e. allowing many differently sequenced IPs to bind and prime, followed by identical or distinct booster sequence units.

A further possibility for detecting or monitoring the progression of the initiated GMA reaction is by monitoring DNA polymerase activity. Since GMA results in the continuous re-extension (i.e. polymerisation) of DNA fragments on a template, there is continuous incorporation of dNMP into the newly synthesised DNA and release of a pyrophosphate moiety (PPi) *per* each incorporated nucleotide monophosphate. Therefore, a PPi detection assay (Nyren, P. (1987) Analytical Biochemistry, 167, 235-238) can be used to indirectly detect or monitor GMA activity.

In a GMA reaction using the precursor nucleotides dATP, dCTP, dGTP, dTTP in addition to a modified precursor nucleotide such as dUTP, the displaced fragments which are generated are of multiple sizes according to the permutations of dUTP *versus* dTTP incorporation into the newly synthesised complementary strand opposite the A residues in the template strand. A suitable IP binding site on a booster template in such a case is a sequence that is identical to a section of the initial template which is 5' (with respect to the template) of where the IP initially primed. In a sequence of DNA, a dUTP or a dTTP would be expected to be incorporated on average every fourth base since an A residue would be expected to occur at any given position in the template with a frequency of 0.25. Thus the average expected size of a displaced fragment in a GMA reaction, using dUTP in place of dTTP, would be three nucleotides. Whereas, using a ratio of dUTP to dTTP where the DNA polymerase inserts either of the two dNTPs with a 0.5 probability, the size of generated displaced fragments will range from a minimal size of three nucleotides to larger sizes where the frequency of generation of any displaced fragment decreases as the size of the displaced fragment gets larger. However, the larger the displaced fragment, the more stringent can be the hybridisation between the displaced fragment acting as an IP and the IP binding site. Thus an IP binding site on the booster template that is identical to a sequence 5' of the IP binding site on the original template nucleic acid is desirable. For example, if a displaced fragment is selected so that it has 10 positions within a 40 nucleotide segment where a dUTP or dTTP could be incorporated, an IP binding site on the booster template, complementary to the 40 nucleotides of the displaced DNA is suitable.

For detection of SNPs or mutations, the IP that primes the template nucleic acid can be positioned 3' from the chosen SNPs location with respect to the template strand, so that one permutation of the displaced fragment generated has its 3' terminus defined by the SNP site. In such a case, the presence of the SNP on the template nucleic acid leads to the generation of a displaced fragment with a unique 3'OH terminus that is not created if the SNP site is absent. The IP can be chosen so that in a GMA reaction, using a mixture of a normal and modified precursor such as dTTP and dUTP, a displaced fragment is generated of a suitable size so as to permit its hybridisation to the IP binding site on the booster template under stringent conditions. Furthermore, the IP binding site on the booster template can be designed so that it can only be primed by that displaced fragment with the unique 3'OH terminus as defined by the SNP site and the status of that site. This can be achieved by designing the booster template so that the key adenine residue supporting the GMA is sufficiently closely located 5' on the booster template so that, the 3'end of the displaced fragment generated with a 3'OH terminus defined by the next position of dUTP incorporation past the SNP site on the original template, is opposite or 5' of the key adenine residue on the booster template. Under stringent hybridisation conditions, displaced fragments where the 3'OH terminus is generated from a site upstream of the SNP site (with respect to the complement of that strand that acts as template) will not prime the booster template as they will not hybridise to the IP binding site.

According to a further embodiment of the invention an IP binding site may be degenerate so that it can serve as a binding site for many different initiating primers. Two or more booster templates can be included in a GMA reaction so that the cBS generated from the first booster template can serve as an initiating primer for the second booster template. This has the added advantage that the second booster template can be identical for multiple different GMA reactions and thus serve as a universal booster sequence. This facilitates a single streamlined process for detection and signal amplification using a single end point booster template. Typically the end point booster template will be designed and synthesised so that it serves directly or indirectly as the reporter for the GMA.

The IP in a GMA reaction may displace a downstream extended primer that serves as an IP in a subsequent GMA reaction. This has important uses for SNP and mutation detection. A primer may be placed sufficiently close to the SNP site so that the first modified precursor incorporated is at, or distal to, the SNP site. Thus the primer is extended to a different length depending on whether or not a SNP is present at the site. It is desirable to generate multiple copies of the differentially extended primer for subsequent characterisation by any one of several means including its ability to function as an IP in a subsequent GMA on a booster template. Multiple copies of the differentially extended primer may be obtained by a thermocycling process as described for the polymerase chain reaction. Alternatively, the differentially extended primer may be repeatedly displaced by initiating a GMA reaction 5' of this primer (i.e. further 3' on the template strand). This is achieved using an IP which hybridises 5' of the primer located proximal to the SNP site and this IP initiates a GMA reaction. The downstream primer will be extended in the same reaction in each cycle but will also be displaced in each cycle. Once displaced, fresh primer can hybridise and be extended.

Displaced fragments generated from template nucleic acids, and complementary booster sequences generated from the booster template may be detected in a 5' nuclease assay. Typically, the 5' nuclease assay is an assay that detects a specific nucleic acid by its ability to serve as an initiating primer for DNA synthesis on a template using a DNA polymerase with a 5'-3' exonuclease activity leading to degradation of a probe which is annealed downstream on the template. This degradation is based on the presence of a 5'-3' exonuclease activity in the polymerase used in the reaction. The typical probe is an oligonucleotide bearing both a reporter fluorescent dye and a quencher dye in close proximity. An increase in fluorescent intensity results when the reporter and quencher are separated / detached from each other through degradation of the probe. Thus an increase in fluorescence indicates that the probe has hybridised to the template and has been degraded by the 5' to 3' exonuclease activity of the DNA polymerase as it extends the initiating primer on the template nucleic acid.

A variation of this assay uses an approach whereby the probe is part of the template but is complementary to a section of template. This results in the probe forming a stem with part of the template by base pairing with its complement on the template nucleic acid, effectively producing a double stranded stem with one free 5' end and one looped end.

Alternatively, a self complementary probe with a reporter at one end and a quencher at the other end may be used. In this case, the probe forms a stem and loop through base pairing bringing the reporter close to the quencher so that fluorescence is quenched. Denaturation of the stem loop structure in the presence of a fully or partially complementary displaced fragment or cBS permits hybridisation between the probe and the displaced fragment or cBS. This renders the probe double stranded and increases the distance between the reporter and quencher causing an increase in fluorescent intensity.

Adaptation of the GMA for use with a linked reporter - quencher provides a unique booster template that permits simultaneous IP detection and signal amplification. Essentially, the booster template is designed with an IP binding site followed by a self complementary sequence that brings a linked reporter and quencher into close proximity through formation of a double stranded stem loop structure. The IP binding site and the sequence forming the stem - loop structure are devoid of the base complementary to the modified base in the GMA reaction. The IP binding site is 3' of the stem - loop sequence and may comprise a sequence which is complementary to an initiating primer and / or a sequence which is complementary to a cBS. One or more bases complementary to the modified base in the GMA reaction are present 5' of the stem - loop and are followed by another complementary sequence to a cBS. When an initiating primer is extended on the reporter-quencher booster template, the net effect is that the IP binding site and stem - loop section of the booster template is linearised and becomes double stranded during GMA through strand displacement and remains double stranded. The signal is amplified through the synthesis of further cBSs at the 5' end of the booster template which in turn serve as initiating primers for any non-primed booster template in the GMA reaction. When the stem-loop is linearised and becomes double stranded, the fluorescence intensity increases and measurement of the fluorescences functions as a measure of the level on IP in the reaction.

An alternative method of signal detection in accordance with the invention involves designing the booster template so that part or all of the displaced fragment is self complementary either within itself or between other copies of itself and forms double stranded DNA. The double stranded DNA can then be detected readily by binding of double strand specific probes such as SYBR green.

The booster template may be circular. Such a circular booster template provides a means for the extending DNA polymerase to proceed continuously in a rolling circle replication format. There are multiple variations that can be used with a circular booster template. In its simplest form, the circular booster template may serve as a template for rolling circle amplification where an IP may serve as the initiator of DNA replication on the circle. In such a case, the DNA is continuously synthesised until the polymerase or the DNA precursors become limiting. Inclusion of such a booster template in a GMA reaction requires that the booster template is designed so that it does not contain any residues complementary to the modified precursor nucleotide in the GMA reaction.

Alternatively, the booster template may be designed with one or more IP binding sites followed by a residue supporting incorporation of a modified precursor on to the extending IP during GMA. This may be further followed by one or more booster sequences. The booster sequences may be preceded and followed by one or more bases that are complementary to the modified base in the GMA reaction. The complement of the booster sequence, when generated, serves as an IP for any non-primed booster templates. Priming of subsequent booster templates provides additional IP for additional non-primed booster templates and the reaction continues until all of the booster templates are primed and replication continues until one of the reagents becomes limiting. The net effect of this method is that a linear copy of all or part of the booster template is generated in each cycle. Inclusion of a second complementary booster template with a self-priming booster sequence provides a means of generating multiple linear copies of the booster template. The linear copies of the two booster templates will then hybridise to each other generating double stranded DNA which can be detected by several means, including double strand specific DNA binding agents such as SYBR green.

A single booster template may also be designed so that it produces cBSs that base pair with each other thus generating double stranded DNA. The booster template is particularly suitable for this application since regions of self complementarity within a DNA circle can be primed without denaturation by IPs hybridising to IP binding sites which are not in the self complementary region. In contrast, denaturation of fully double stranded linear complementary DNA is necessary to allow an IP access an IP binding site.

In another embodiment of the present invention the booster or secondary template can be immobilised on a solid support, for example, on a micro- or macro-array or DNA 'chip'. Multiple different booster or secondary templates can be immobilised on a DNA chip which can then be used to characterise multiple different nucleic acids and multiple different GMA reactions in a high throughput manner. In a preferred embodiment one attaches the secondary templates *via* their 3' termini. This makes the otherwise reactive 3'OH group inaccessible, therefore it cannot be extended by a polymerase and acts only as a template, thereby reducing any background non-specific extension.

Another embodiment of the present invention provides for the use of GMA in DNA computing. It is now increasingly appreciated that DNA, by virtue of its intrinsic physical and chemical properties, may provide an avenue for the computation of solutions to difficult mathematical tasks. It will be appreciated that by designing initiating primers, templates and/or booster templates to act as individual or combined pieces of information, be it problems and/or solutions, the solutions to mathematical problems can be computed using the GMA reaction. Preferably, the IPs and templates are artificially synthesised. It will also be appreciated that the use of GMA in DNA computing permits simultaneous operations and parallel searches and can give rise to a complete set of potential solutions.

Accordingly, the invention provides use of a method as hereinbefore described in DNA computing and/or as a "tool" in DNA computing, referred to herein collectively as DNA computing.

The invention will be further illustrated by the following Examples.

### Modes for Carrying Out the Invention

Various enzymes were used in the following Examples. Some of these enzymes were available commercially while others were purified from over-expression in *E. coli* strains as described below.

### Thermotoga maritima UDG (TmaUDG)

The open reading frame for the TmaUDG protein was amplified from *T. maritima* genomic DNA by PCR. The PCR product was inserted into the pBAD-TOPO over-expression vector according to the manufacturer's instructions (In Vitrogen). Competent TOP-10 *E. coli* (In Vitrogen) were then transformed with the construct by heat shock, according to the manufacturer's instructions. Cells containing the pBAD-TOPO/TmaUDG construct were grown to an OD₆₀₀ of 0.6 (1000mL) and over-expression was induced by arabinose added to a final concentration of 0.2%. After incubation at 37°C for 4hours the cells were lysed and the fusion protein was purified by immobilised metal affinity chromatography using ProBond resin (Invitrogen) according to the manufacturer's instructions. A 15mL fraction containing the eluted protein as judged by SDS-polyacrylamide gel electrophoresis was collected. The fraction was then further purified by ion-exchange chromatography using Mono-S resin and 0.5mL of the most concentrated eluted protein fraction was collected and stored after addition of glycerol to 50% at -20°C until required.

### Thermotoga maritima Endonuclease IV (TmaEndoIV)

The open reading frame for the TmaEndoIV protein was amplified from *T. maritima* genomic DNA by PCR. The PCR product was inserted into the pBAD-TOPO over-expression vector according to the manufacturer's instructions (InVitrogen). Competent TOP-10 *E. coli* (In Vitrogen) were then transformed with the construct by heat shock, according to the manufacturer's instructions. Cells containing the pBAD-TOPO/TmaEndoIV construct were grown to an OD₆₀₀ of 0.6 (1000mL) and over-expression was induced by arabinose added to a final concentration of 0.2%. After incubation at 37°C for 4hours the cells were lysed and the fusion protein was purified by immobilised metal affinity chromatography using ProBond resin (Invitrogen) according to the manufacturer's instructions. A 15mL fraction containing the eluted protein as judged by SDS-polyacrylamide gel electrophoresis was collected. The fraction was then further purified by ion-exchange chromatography using Mono-S resin and 0.5mL of the most concentrated eluted protein fraction was collected and stored after addition of glycerol to 50% at -20°C until required.

### Thermotoga maritima Endonuclease V (TmaEndoV)

The open reading frame for the TmaEndoV protein was amplified from *T. maritima* genomic DNA by PCR. The PCR product was inserted into the pBAD-TOPO over-expression vector according to the manufacturer's instructions (InVitrogen). Competent TOP-10 *E. coli* (In Vitrogen) were then transformed with the construct by heat shock, according to the manufacturer's instructions. Cells containing the pBAD-TOPO/TmaEndoV construct were grown to an OD₆₀₀ of 0.5 (1000mL) and over-expression was induced by arabinose added to a final concentration of 0.2%. After incubation at 37°C for 8hours the cells were lysed and the fusion protein was purified by immobilised metal affinity chromatography using ProBond resin (Invitrogen) according to the manufacturer's instructions. 15 x 1mL fractions containing the eluted protein as judged by SDS-polyacrylamide gel electrophoresis were collected and pooled. The pooled fractions were then further purified by ion-exchange chromatography using Mono-S resin and 0.5mL of the most concentrated eluted protein fraction was collected and stored after addition of glycerol to 50% at -20°C until required.

### E. coli Endonuclease IV (EcoEndoIV)

The open reading frame for the EcoEndoIV protein was amplified from *E. coli* genomic DNA by PCR. The PCR product was inserted into the pBAD-TOPO over-expression vector according to the manufacturer's instructions (InVitrogen). Competent TOP-10 *E. coli* (InVitrogen) were then transformed with the construct by heat shock, according to the manufacturer's instructions. Cells containing the pBAD-TOPO/EcoEndoIV construct were grown to an OD₆₀₀ of 0.6 (1000mL) and over-expression was induced by arabinose added to a final concentration of 0.2%.

After incubation at 37°C for 4hours the cells were lysed and the fusion protein was purified by immobilised metal affinity chromatography using ProBond resin (Invitrogen) according to the manufacturer's instructions. A 15mL fraction containing the eluted protein as judged by SDS-polyacrylamide gel electrophoresis was collected. The pooled fractions were then further purified by ion-exchange chromatography using Mono-S resin and 0.5mL of the most concentrated eluted protein fraction was collected and stored after addition of glycerol to 50% at -20°C until required.

### Archaeoglobus fulgidus UDG-Thioredoxin (AfUDG-Thio)

The open reading frame for the AfUDG protein was amplified from *A. fulgidus* genomic DNA by PCR. The PCR product was inserted into the pBAD-TOPO ThioFusion over-expression vector according to the manufacturer's instructions (In Vitrogen). Competent TOP-10 *E. coli* (In Vitrogen) were then transformed with the construct by heat shock, according to the manufacturer's instructions. Cells containing the pBAD-TOPO-ThioFusion/AfUDG construct were grown to an OD₆₀₀ of 0.6 (2000mL) and over-expression was induced by arabinose added to a final concentration of 0.2%. After incubation at 37°C for 4hours the cells were lysed and the fusion protein was purified by immobilised metal affinity chromatography using ProBond resin (Invitrogen) according to the manufacturer's instructions. A 15mL fraction containing the eluted protein as judged by SDS-polyacrylamide gel electrophoresis was collected and stored at 4°C until required.

### Example 1

The method according to the invention was used to cyclically extend a 25-mer oligonucleotide (Initiating Primer-IP), which was complementary to a region of an 80-mer oligonucleotide (Template nucleic acid) which served as the template for the polymerisation reaction. The complementary region extended from 10 bases from the 3' end of the 80-mer to 35 bases from the 3' end of the 80-mer. The region of the 80-mer 5' to this IP complementary region (IP binding site) was designed to contain a number of A residues which upon cyclical extension of the 25-mer would lead to the production of DNA fragments of discrete sizes following the GMA reaction. The 80-mer was also designed so that a specific number of G residues lay between each A residue. This was to allow labelling, by incorporation of α³²P-dCTP, of the displaced downstream DNA fragments produced during the reaction. A flow diagram of the method is shown in Fig. 1. Both oligonucleotides were synthesised artificially and purified by excision from a polyacrylamide gel after electrophoresis. The objective was to determine if the 25-mer annealed to the 80-mer and could be cyclically/repeatedly extended by the method according to the invention, producing multiple copies of labelled single stranded DNA fragments (displaced fragments).

Before addition of the enzymes, the reactions contained 100fmole of each of the oligonucleotides, 10mM Tris-HCl (pH7.5), 5mM MgCl₂, 7mM dithiothreitol, 0.2mM each of dATP, dGTP and dUTP, 0.02mM dCTP and 0.2µL α³²PdCTP (10mCi/ml, ~800Ci/mmol) in a total of 17µL. This mixture was overlaid with mineral oil and heated to 95°C for 2min. The reaction temperature was then dropped to 37°C and held at this temperature. Five units of Klenow Fragment (3' → 5' exo⁻), 1 unit of *E. coli* Uracil DNA Glycosylase and 2 units of either *E*. *coli* Endonuclease IV or 2µL *Thermotoga maritima* Endonuclease IV were then added, in that order, bringing the final reaction volume to 20µL. In the case of the control reactions, which contained no Endonuclease IV and the control reactions that contained neither Endonuclease IV nor Uracil DNA Glycosylase, water was added to bring the final volume to 20µL. 5µL samples were taken from the reactions at 30min intervals after the addition of the Endonuclease IV. NaOH was added to the samples to a final concentration of 50mM and then heated to 95°C for 15min. An equal volume of formamide loading dye (98% formamide, 0.025% Bromophenol blue, 0.025% Xylene cyanol) was then added. The samples were then loaded onto a 20% denaturing (7M urea) polyacrylamide gel and electrophoresis was carried out for size analysis of the DNA fragments. Following electrophoresis, the gel was exposed to a phosphor screen and the image was subsequently scanned by a Storm (Trade Mark) 860.

Analysis of the image showed that the reactions which contained Endonuclease IV contained a number of labelled DNA fragments of different sizes shorter than 70 bases, which increased in number with time, and which did not appear in the control reactions which lacked the Endonuclease IV, or in the control reaction which lacked both Endonuclease IV and Uracil DNA Glycosylase.

It was noted that the *Thermatoga maritima* Endonuclease IV preparations contained an intrinsic and/or contaminating 3' to 5' exonuclease activity. This lead to non-specific background amplification during a GMA reaction. To eliminate this activity and non-specific amplification, the Endonuclease IV enzyme was heat treated prior to its use in the GMA reaction. The stock of Endonuclease IV enzyme was heated to 90°C for 10 min, then put on ice for ≥ 5 min and subsequently centrifuged for ≥ 5 min. The *E. coli* Endonuclease IV preparations were also observed to contain such an activity and the preparations also required inactivation of the exonuclease activity prior to use.

### Example 2

The method according to the invention was used to cyclically extend a 41-mer oligonucleotide (Initiating Primer-IP) which was self-complementary at its 3' end, i.e. palindromic, and therefore served as the template for itself for the GMA reaction. The complementary region extended for 20 bases from the 3' end of the 41-mer and this section of the oligonucleotide esssentially acted as the IP. The region of the 41-mer 5' to this IP complementary region (IP binding site) was designed to contain one A residue following the IP binding site, which upon cyclical extension of the 41-mer would lead to the production of a discrete DNA fragment of 20 nucleotides following the GMA reaction. The 41-mer was also designed so that it contained a number of G residues. This was to allow labelling, by incorporation of α³²P-dCTP, of the displaced downstream DNA fragments produced during the reaction (20-mer). The oligonucleotide was synthesised artificially and purified by excision from a polyacrylamide gel after electrophoresis. The objective was to determine if the 41-mer annealed to itself and could be cyclically/repeatedly extended by the method according to the invention, producing multiple copies of the labelled single stranded 20-mer DNA fragment (displaced fragment).

Before addition of the enzymes, the reactions contained 100fmole of the 41-mer oligonucleotide, 10mM Tris-HCl (pH7.5), 5mM MgCl₂, 7mM dithiothreitol, 0.2mM each of dATP, dGTP and dUTP, 0.02mM dCTP and 0.2µL α³²PdCTP (10mCi/ml, ~800Ci/mmol) in a total of 17µL. This mixture was overlaid with mineral oil and heated to 95°C for 2min. The reaction temperature was then dropped to 37°C and held at this temperature. Five units of Klenow Fragment (3' → 5' exo⁻), 1 unit of *E. coli* Uracil DNA Glycosylase and 2 units of either *E. coli* Endonuclease IV or 2µL *Thermotoga maritima* Endonuclease IV were then added, bringing the final reaction volume to 20µL. In the case of the control reactions which contained no Endonuclease IV and the control reactions which contained neither Endonuclease IV nor Uracil DNA Glycosylase, water was added to bring the final volume to 20µL. The reaction was allowed to proceed for greater than or equal to 30 min. EDTA was added to a final concentration of 10mM to stop the reaction. An equal volume of formamide loading dye (98% formamide, 0.025% Bromophenol blue, 0.025% Xylene cyanol) was then added. The samples were then loaded onto a 20% denaturing (7M urea) polyacrylamide gel and electrophoresis was carried out for size analysis of the DNA fragments. Following electrophoresis, the gel was exposed to a phosphor screen and the image was subsequently scanned by a Storm 860 (Storm is a Trade Mark).

Analysis of the image showed that a detectable quantity of the labelled and amplified 20-mer oligonucleotide was produced in each test reaction, but not in the control reactions.

### Example 3

The method according to the invention was carried out as described in Example 2 with some modifications, as follows:

Before addition of the enzymes the reaction contained 100fmole 41-mer palindromic oligonucleotide, 10mM Tris-HCl (pH 8.3) 10mM KCl, 3mM MgCl₂, 0.2mM each of dATP, dUTP and dGTP, 0.02mM dCTP and α-³²P-dCTP in a volume of 20µL. This mixture was overlaid with mineral oil and heated to 95°C for 2min. The temperature was then dropped to 60°C and held there. 10 units of AmpliTaq (Trade Mark) DNA Polymerase Stoffel Fragment, 2µL units *Thermotoga maritima* UDG and 4 units *E. coli* Endonuclease IV (or water in the case of the control reaction containing no Endonuclease IV) were added to the reaction in that order, to bring the final reaction volume to 25µL. 5µL samples of the reactions were taken at 30min intervals after the addition of the Endonuclease IV. These samples were brought to a final concentration of 10mM EDTA. An equal volume of formamide loading dye (98% formamide, 0.025% Bromophenol blue and 0.025% Xylene cyanol) was then added. Size analysis of the DNA samples was carried out as in Example 1.

Analysis of the image showed that the reactions containing Exonuclease IV contained large amounts of labelled DNA, which was absent from the control reactions which lacked the Endonuclease IV. The amount of DNA in the test reactions increased with time reaching a peak at 90min from the addition of the Endonuclease IV.

### Example 4

The method according to the invention was carried out as described in Example 2 with some modifications, as follows

Before addition of the enzymes the reaction contained 100fmole of the 41mer oligonucleotide, 10mM Tris-HCl (pH 8.3) 10mM KCl, 3mM MgCl₂, 0.2mM each of dATP, dUTP and dGTP, 0.02mM dCTP and 0.2µL α-³²P-dCTP (10mCi/ml, ~800Ci/mmol) in a volume of 20µL. This mixture was overlaid with mineral oil and heated to 95°C for 2min. The temperature was then dropped to 70°C and held there. 10 units of AmpliTaq DNA Polymerase Stoffel Fragment, 2µL AfUDG-Thio and 2µL EcoEndoIV (or water in the case of the control reaction containing no Endonuclease IV) were added to the reaction in that order, to bring the final reaction volume to 25µL. The reaction was terminated after 60min by the addition of EDTA to a final concentration of 10mM. An equal volume of formamide loading dye (98% formamide, 0.025% Bromophenol blue and 0.025% Xylene cyanol) was then added. Size analysis of the DNA samples was carried out as in Example 1. Following electrophoresis, the gel was exposed to a phosphor screen and the image was detected using a Storm (Trade Mark) 860 phosphoimager.

Analysis of the image showed that the reactions containing Exonuclease IV contained large amounts of the expected labelled 20mer and to a lesser extent, some smaller fragments, which were absent from the control reactions in the absence of added Endonuclease IV.

### Example 5

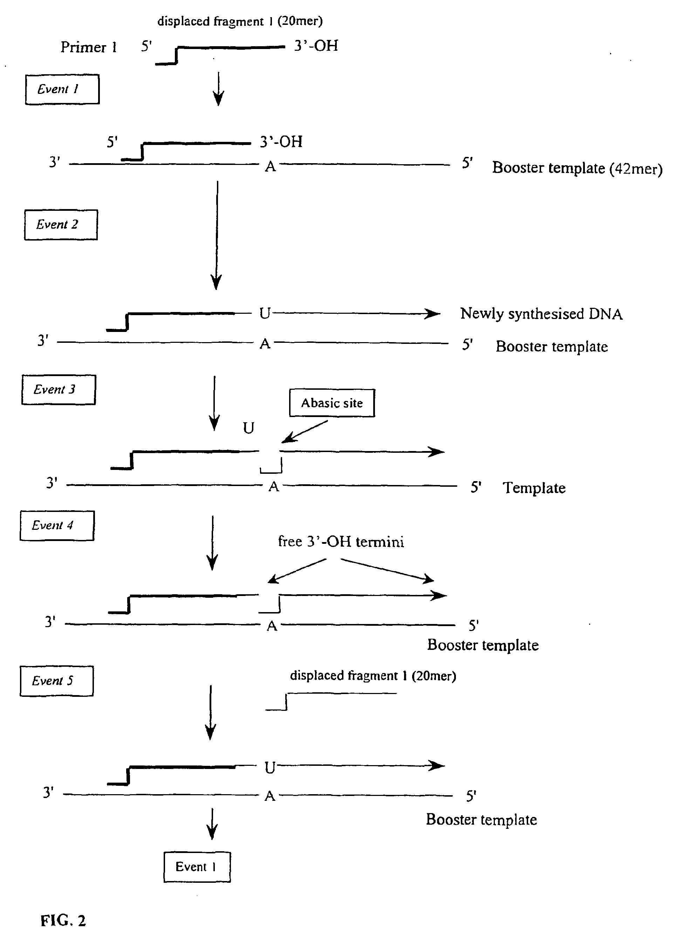

The method according to the invention was used to detect primer extension from attomole quantities of the 41-mer palindromic oligonucleotide. Uracil DNA Glycosylase digestion followed by Endonuclease IV digestion of the product formed by the polymerase catalysed extension of the 41-mer oligonucleotide using itself as template yields as one of its products a 20-mer single stranded oligonucleotide (i.e. displaced fragment which can subsequently act as an IP). This oligonucleotide acts as a secondary primer. In this example the method according to the invention allows the detection of the cyclical extension of the 20-mer oligonucleotide through a feedback loop reaction. A 42-mer oligonucleotide (booster template) was designed as schematically represented in Fig. 2. The complementary sequence of the 20-mer secondary primer/displaced fragment is repeated twice in this 42-mer. The repeated sequences are separated (reading 5' to 3') by an AT sequence. This oligonucleotide was synthesised and purified in the same way as the 41-mer palindromic oligonucleotide. This 42-mer was included in large excess in the reaction. Once the 20-mer secondary primer has been produced as described above, it can then anneal to the 42-mer oligonucleotide and, by the method according to the invention, be itself cyclically extended, producing as a result numerous copies of itself. These newly produced 20-mer secondary primers can then themselves anneal to other copies of the 42-mer oligonucleotide initiating new rounds of cyclical extension and so on. The objective was to detect quantities of the 41-mer palindromic oligonucleotide, normally too small to detect as described in Example 2, by the production of detectable quantities of the 20-mer secondary primer through the feedback loop application of the method according to the invention described above.

The reactions described in Example 2 were repeated in triplicate except for the following modifications. The 42-mer described above was included in each reaction to a final concentrations of 40ng/reaction. The concentration of the 41-mer palindromic oligonucleotide was 1.0 fmole, 10⁻² fmole or 10⁻⁴ fmole *per* reaction in the different sets of control (no Endonuclease IV included in the reaction) and test reactions (Endonuclease IV included in the reaction). The reactions were allowed to run for 2 hours before the addition of the NaOH to a final concentration of 50mM. The labelled DNA fragment content of the reactions was analysed by analysis of the equivalent of 5µL of the original reactions (before addition of the NaOH and formamide loading dye) as described in Example 1.

Analysis of the image showed that a detectable quantity of the labelled 20-mer oligonucleotide was produced in each test reaction, but not in the control reactions.

A similar result was observed when the above experiment was repeated using the thermostable enzymes, DNA Stoffel Fragment, *Thermatoga maritima* UDG and *Thermatoga maritima* Endonclease IV. To avoid any non-specific amplification arising from the self- priming of the booster template, the 3' end of the booster usually contains a blocking group which prevents extension by DNA polymerase. The blocking group used on the 42-mer booster described above was a dideoxycytosine nucleotide.

### Example 6

The method according to the invention was used to cyclically extend a self-annealing 41mer. This reaction produced a 20mer that could then act as an initiation primer on a 42mer (booster) with a blocked 3' terminus (the block used was an inverted cytidine nucleotide). The booster was designed such that this secondary reaction would also produce the same 20mer by cyclical extension (see Fig. 2). These 20mers could then prime further extension on other booster molecules, amplifying the signal from the original reaction on the 41mer. The 41mer and 42mer booster were designed to allow the incorporation of radio-labelled α³²PdCTP. Reactions contained either (1) 100fmole of the 41mer, (2) 1fmole of the 41mer, (3) 100fmole of booster or (4) 1fmole of the 41mer and 100fmole of a booster in Taq Stoffel Fragment buffer (10mM Tris-HCl, 10mM KCl, pH8.3) supplemented with 3mM MgCl₂, 0.2mM dATP, 0.2mMdGTP, 0.2mM dUTP and 0.02mM and 0.2uL α³²PdCTP (10mCi/ml, ~800Ci/mM). This reaction mix was overlaid with oil and incubated at 95°C for 2min before being brought to and maintained at 70°C. 10U Taq Stoffel Fragment, 2µL AfUDG-Thio and 2µL EcoEndoIV were then added in that order, bringing the final reaction volume to 25µL. The reaction was incubated at 70°C for 60min and then terminated by the addition of EDTA to a final concentration of 10mM. An equal volume of 98% formamide loading buffer (containing 0.025% Bromophenol blue, 0.025% Xylene cyanol) was added and samples were analysed by denaturing 20% polyacrylamide gel electrophoresis. Following electrophoresis, the gel was exposed to a phosphor screen and the image was detected using a Storm (Trade Mark) 860 phosphoimager.

Analysis of the image showed that in reaction (1) there was production of a 20mer labelled DNA product, and to a lesser extent a ~60mer and a number of products smaller than the 20mer. The same product was produced in reaction (2) but to a far lesser extent. There was no labelled reaction product in reaction (3) however in reaction (4) a labelled 20mer was produced which was more intense than that produced in reaction (2) as well as to a lesser extent a number of labelled products smaller than the 20mer. It was observed that the use of inverted nucleotides as 3' terminal blocks was more effective than use of dideoxynucleotides as terminal blocks.

### Example 7

The method according to the invention was used to cyclically extend a 41-mer oligonucleotide (Initiating Primer-IP), which was self-complementary at its 3' end, i.e. palindromic, and therefore served as the template for itself for the GMA reaction. The complementary region extended for 20 bases from the 3' end of the 41-mer and this section of the oligonucleotide essentially acted as the IP. The region of the 41-mer 5' to this IP complementary region (IP binding site) was designed to contain one A residue following the IP binding site, which upon cyclical extension of the 41-mer would lead to the production of a discrete DNA fragment of 20 nucleotides following the GMA reaction. The 41-mer was also designed so that it contained a number of G residues. This was to allow labelling, by incorporation of α³²P-dCTP, of the displaced downstream DNA fragments produced during the reaction (20-mer). The oligonucleotide was synthesised artificially and purified by excision from a polyacrylamide gel after electrophoresis. The objective was to determine if the 41-mer annealed to itself and could be cyclically/repeatedly extended by the method according to the invention with *E. coli* Exonuclease III in place of *E. coli* endonuclease IV, producing multiple copies of the labelled single stranded 20-mer DNA fragment (displaced fragment). Before addition of the enzymes, the reactions contained 100fmole of the 41-mer oligonucleotide, 10mM Tris-HCl (pH7.5), 5mM MgCl₂, 7mM dithiothreitol, 0.2mM each of dATP, dGTP and dUTP, 0.02mM dCTP and 0.2µL α³²PdCTP (10mCi/ml, ~800Ci/mmol) in a total of 22µL. This mixture was overlaid with mineral oil and heated to 95°C for 2min. The reaction temperature was then dropped to 37°C and held at this temperature. Five units of Klenow Fragment (3' → 5' exo⁻), 10 units of *E*. *coli* Uracil DNA Glycosylase and 0.1 units of *E. coli* exonuclease III were then added, bringing the final reaction volume to 25µL. In the case of the control reactions which contained no Exonuclease III, water was added to bring the final volume to 25µL. The reaction was allowed to proceed for greater than or equal to 3 0 min. EDTA was added to a final concentration of 10mM to stop the reaction. An equal volume of formamide loading dye (98% formamide, 0.025% Bromophenol blue, 0.025% Xylene cyanol) was then added. The samples were then loaded onto a 20% denaturing (7M urea) polyacrylamide gel and electrophoresis was carried out for size analysis of the DNA fragments. Following electrophoresis, the gel was exposed to a phosphor screen and the image was detected using a Storm (Trade Mark) 860 phosphoimager.

Analysis of the image showed that a detectable quantity of the labelled 20-mer oligonucleotide was produced in each test reaction, but not in the control reactions.

### Example 8

The method according to the invention was used to cyclically extend a 21-mer oligonucleotide (Initiating Primer-IP), which was complementary to a region of a 40-mer oligonucleotide (template nucleic acid) which served as the template for the polymerisation reaction. The complementary region extended from the 3' end of the 40-mer. The region of the 40-mer 5' to this IP complementary region (IP binding site) was designed to contain a number of C residues which upon cyclical extension of the extended 21-mer would lead to the production of DNA fragments of discrete sizes following the GMA reaction. The 40-mer was also designed so that a number of G residues lay between each A residue. This was to allow labelling, by incorporation of α³²P-dCTP, of the displaced downstream DNA fragments produced during the reaction. Both oligonucleotides were synthesised artificially and purified by excision from a polyacrylamide gel after electrophoresis. The objective was to determine if the 21-mer annealed to the 40-mer and could be cyclically/repeatedly extended by the method according to the invention, producing multiple copies of labelled single stranded DNA fragments (displaced fragments). Before addition of the enzymes, the reactions contained 100fmole of each of the oligonucleotides, 10mM Tris-HCl (pH7.5), 6mM MgCl₂, 10mM KCl, 0.2mM each of dATP, dITP and dTTP, 0.02mM dCTP and 0.2µL α³²PdCTP (10mCi/ml, ~800Ci/mmol) in a total of 22µL. This mixture was overlaid with mineral oil and heated to 95°C for 2min. The reaction temperature was then dropped to 70°C and held at this temperature. Ten units of Taq polymerase Stoffel Fragement, and 800pg of TmaEndoV was then added, in that order, bringing the final reaction volume to 25µL. In the case of the control reactions, which contained no Endonuclease V, water was added to bring the final volume to 25µL. Reactions were terminated by the addition of EDTA to a final concentration of 10mM. One half volume of formamide loading dye (98% formamide, 0.025% Bromophenol blue, 0.025% Xylene cyanol) was then added and the reaction products denatured by heating to 95°C for 5min. The samples were then loaded onto a 20% denaturing (7M urea) polyacrylamide gel and electrophoresis was carried out for size analysis of the DNA fragments. Following electrophoresis, the gel was exposed to a phosphor screen and the image was detected using a Storm (Trade Mark) 860 phosphoimager.

Analysis of the image showed that the reactions, which contained Endonuclease V, contained a number of labelled DNA fragments of different sizes which were delineated by the relevant position of dITP incorporation in the extension, and which increased in number with time, and which did not appear in the control reactions that lacked the Endonuclease V.

## Claims

1. A method for the amplification of a template nucleic acid, which comprises simultaneously carrying out the steps of:
i) reacting a nucleic acid primer with said template nucleic acid, normal DNA precursor nucleotides, at least one modified DNA precursor nucleotide and a DNA polymerase so as to obtain an extended nucleic acid primer, said nucleic acid primer remaining bound to said template;
ii) cleaving the modified base-containing extended nucleic acid primer so as to generate a free 3'-OH terminus that is extendible by said DNA polymerase; and
iii) repeating steps i) and ii) on DNA fragments thereby generated.

2. A method according to Claim 1, wherein the modified base-containing extended nucleic acid primer is cleaved by a 3'-endonuclease.

3. A method according to Claim 2, wherein the 3'-endonuclease is Endonuclease V from *E*. *coli.*

4. A method according to Claim 1, which comprises the steps of :
i) reacting a nucleic acid primer with said template nucleic acid, normal DNA precursor nucleotides, at least one modified DNA precursor nucleotide which is a substrate for a DNA glycosylase, and a DNA polymerase so as to obtain an extended nucleic acid primer, said nucleic acid primer remaining bound to said template;
ii) excising the modified base of the modified DNA precursor nucleotide from the extended nucleic acid primer by means of a DNA glycosylase so as to generate an abasic site;
iii) cleaving the extended nucleic acid primer at the abasic site so as to generate a free 3'-OH terminus that is extendible by said DNA polymerase; and
iv) repeating steps i)-iii) on DNA fragments thereby generated.

5. A method according to any preceding claim, wherein the template nucleic acid is DNA.

6. A method according to any preceding claim, wherein the nucleic acid primer is a DNA primer.

7. A method according to any one of Claims 1-6, wherein the DNA precursor nucleotides are selected from dATP, dCTP, dGTP and dTTP.

8. A method according to any preceding claim, wherein the DNA polymerase has strand displacement activity.

9. A method according to any preceding claim, wherein the modified nucleic acid precursor is dUTP.

10. A method according to any one of Claims 4-9, wherein the DNA glycosylase is uracil DNA-glycosylase (UDG).

11. A method according to any one of Claims 4-10, wherein the extended nucleic acid is cleaved at the abasic site by means of an enzyme which cleaves at a nucleic acid abasic site.

12. A method according to Claim 11, wherein the enzyme is an AP endonuclease.

13. A method according to any preceding claim, wherein the modified precursor nucleotide partially replaces one of the normal precursor nucleotides.

14. A method according to any one of Claims 1-3, 5-9 and 13, wherein steps i) and ii) continue in a cyclical manner until one of the reagents becomes limiting.

15. A method according to any one of Claims 4-13, wherein steps i)-iii) continue in a cyclical manner until one of the reagents becomes limiting.

16. A method according to any preceding claim, which is carried out under isothermal conditions.

17. A method according to any preceding claim, which results in the accumulation of displaced single stranded downstream fragments of nucleic acid specified by the locations of modified bases in a complementary nucleic acid strand.

18. A method according to any preceding claim for generating multiple copies of discrete single stranded primers downstream of an initiating nucleic acid primer.

19. A method according to Claim 17 or 18, wherein the displaced downstream fragments are extended in a secondary reaction.

20. A method according to any one of Claims 17-19, wherein the displaced downstream fragments are extended on a secondary template nucleic acid.

21. A method according to any one of Claims 17-20, wherein multiple secondary templates are immobilised on a DNA chip.

22. A method according to any preceding claim for use in detection diagnostics.

23. A method according to Claim 22, which is used in the detection of pathogens.

24. A method according to Claim 22, which is used in the detection of the presence or absence of mutations.

25. A method according to Claim 22, which is used in the detection of polymorphisms.

26. A method according to any preceding claim for the quantification of the level of a nucleic acid in a sample.

27. A method according to any preceding claim for use in signal amplification from any nucleic acid that can function as a primer or template.

28. A method according to any preceding claim, for use in DNA computing.

## Patentansprüche

1. Verfahren zur Amplifikation einer Template-Nukleinsäure, das das gleichzeitige Ausführen der folgenden Schritte umfasst:
(i) Umsetzen eines Nukleinsäureprimers mit der Template-Nukleinsäure, normalen DNA-Vorläufer-Nukleotiden, mindestens einem modifizierten DNA-Vorläufer-Nukleotid und einer DNA-Polymerase, um einen verlängerten Nukleinsäureprimer zu erhalten, wobei der Nukleinsäureprimer an das Template gebunden bleibt;
(ii) Spalten des verlängerten Nukleinsäureprimers, der die modifizierte Base enthält, um einen freien 3'-OH-Terminus zu bilden, der durch die DNA-Polymerase verlängerbar ist; und
(iii) Wiederholen der Schritte (i) und (ii) mit den hierdurch gebildeten DNA-Fragmenten.

2. Verfahren nach Anspruch 1, wobei der verlängerte Nukleinsäureprimer, der die modifizierte Base enthält, durch eine 3'-Endonuklease gespalten wird.

3. Verfahren nach Anspruch 2, wobei die 3'-Endonuklease die Endonuklease V aus *E. coli* ist.

4. Verfahren nach Anspruch 1, das die Schritte umfasst:
(i) Umsetzen eines Nukleinsäureprimers mit der Template-Nukleinsäure, normalen DNA-Vorläufer-Nukleotiden, mindestens einem modifizierten DNA-Vorläufer-Nukleotid, das ein Substrat für eine DNA-Glycosylase ist, und einer DNA-Polymerase, um einen verlängerten Nukleinsäureprimer zu erhalten, wobei der Nukleinsäureprimer an das Template gebunden bleibt;
(ii) Ausschneiden der modifizierten Base des modifizierten DNA-Vorläufer-Nukleotids aus dem verlängerten Nukleinsäureprimer mittels einer DNA-Glycosylase, um eine abasische Stelle zu bilden;
(iii) Spalten des verlängerten Nukleinsäureprimers an der abasischen Stelle, um einen freien 3'-OH-Terminus zu bilden, der durch die DNA-Polymerase verlängerbar ist; und
(iv) Wiederholen der Schritte (i) bis (iii) mit den hierdurch gebildeten DNA-Fragmenten.

5. Verfahren nach einem der vorangegangenen Ansprüche, wobei die Template-Nukleinsäure DNA ist.

6. Verfahren nach einem der vorangegangenen Ansprüche, wobei der Nukleinsäureprimer ein DNA-Primer ist.

7. Verfahren nach einem der Ansprüche 1 bis 6, wobei die DNA-Vorläufer-Nukleotide ausgewählt sind aus dATP, dCTP, dGTP und dTTP.

8. Verfahren nach einem der vorangegangenen Ansprüche, wobei die DNA-Polymerase *"strand displacement"*-Aktivität besitzt.

9. Verfahren nach einem der vorangegangenen Ansprüche, wobei der modifizierte Nukleinsäurevorläufer dUTP ist.

10. Verfahren nach einem der Ansprüche 4 bis 9, wobei die DNA-Glycosylase Uracil-DNA-Glycosylase (UDG) ist.

11. Verfahren nach einem der Ansprüche 4 bis 10, wobei die verlängerte Nukleinsäure an der abasischen Stelle mittels eines Enzyms, das eine Nukleinsäure an einer abasischen Stelle spaltet, gespalten wird.

12. Verfahren nach Anspruch 11, wobei das Enzym eine AP-Endonuklease ist.

13. Verfahren nach einem der vorangegangenen Ansprüche, wobei das modifizierte Vorläufer-Nukleotid teilweise eines der normalen Vorläufer-Nukleotide ersetzt.

14. Verfahren nach einem der Ansprüche 1 bis 3, 5 bis 9 und 13, wobei die Schritte (i) und (ii) in einer zyklischen Weise fortgesetzt werden, bis eines der Reagenzien limitierend wird.

15. Verfahren nach einem der Ansprüche 4 bis 13, wobei die Schritte (i) bis (iii) in einer zyklischen Weise fortgesetzt werden, bis eines der Reagenzien limitierend wird.

16. Verfahren nach einem der vorangegangenen Ansprüche, das unter isothermischen Bedingungen durchgeführt wird.

17. Verfahren nach einem der vorangegangenen Ansprüche, das in der Anhäufung von verdrängten (displaced) einzelsträngigen stromabwärts gelegenen Fragmenten der Nukleinsäure resultiert, die durch die Lokalisationen von modifizierten Basen in einem komplementären Nukleinsäurestrang spezifiziert werden.

18. Verfahren nach einem der vorangegangenen Ansprüche zur Bildung von mehreren Kopien diskreter einzelsträngiger Primer, die stromabwärts eines initiierenden Nukleinsäureprimers gelegen sind.

19. Verfahren nach Anspruch 17 oder 18, wobei die verdrängten stromabwärts gelegenen Fragmente in einer Folgereaktion verlängert werden.

20. Verfahren nach einem der Ansprüche 17 bis 19, wobei die verdrängten stromabwärts gelegenen Fragmente auf einer zweiten Template-Nukleinsäure verlängert werden.

21. Verfahren nach einem der Ansprüche 17 bis 20, wobei mehrere Zweittemplates auf einem DNA-Chip immobilisiert sind.

22. Verfahren nach einem vorangegangenen Anspruch zur Verwendung in Nachweis-Diagnostika.

23. Verfahren nach Anspruch 22, das zum Nachweis von Pathogenen verwendet wird.

24. Verfahren nach Anspruch 22, das zum Nachweis der Gegenwart oder Abwesenheit von Mutationen verwendet wird.

25. Verfahren nach Anspruch 22, das zum Nachweis von Polymorphismen verwendet wird.

26. Verfahren nach einem vorangegangenen Anspruch zur Quantifizierung eines Nukleinsäurespiegels in einer Probe.

27. Verfahren nach einem der vorangegangenen Ansprüche zur Verwendung bei der Signalverstärkung einer beliebigen Nukleinsäure, die als ein Primer oder Template wirken kann.

28. Verfahren nach einem vorangegangenen Anspruch zur Verwendung beim DNA-Rechenverfahren.

## Revendications

1. Procédé d'amplification d'une matrice d'acides nucléiques, qui comprend la réalisation simultanée des étapes consistant à :
i) faire réagir une amorce d'acide nucléique avec ladite matrice d'acides nucléiques, des nucléotides précurseurs d'ADN normaux, au moins un nucléotide précurseur d'ADN modifié et un ADN polymérase de manière à obtenir une amorce d'acide nucléique allongée ladite amorce d'acide nucléique restant liée à ladite matrice ;
ii) cliver l'amorce d'acide nucléique allongé contenant la base modifiée de manière à générer une extrémité 3'-OH libre qui puisse être allongée par ledit ADN polymérase ; et
iii) répéter les étapes i) et ii) sur les fragments d'ADN ainsi générés.

2. Procédé selon la revendication 1, dans lequel l'amorce d'acide nucléique allongée contenant la base modifiée est clivée par une endonucléase 3'.

3. Procédé selon la revendication 2, dans lequel l'endonucléase 3' est l'Endonucléase V d'*E*. *coli.*

4. Procédé selon la revendication 1, qui comprend les étapes consistant à :
i) faire réagir une amorce d'acide nucléique avec ladite matrice d'acides nucléiques, des nucléotides précurseurs d'ADN normaux, au moins un nucléotide précurseur d'ADN modifié qui est un substrat pour une ADN glycosylase, et une ADN polymérase de manière à obtenir une amorce d'acide nucléique allongée restant liée à ladite matrice ;
ii) exciser la base modifiée du nucléotide précurseur d'ADN modifié de l'amorce d'acide nucléique allongée au moyen d'une ADN glycosylase de manière à générer un site abasique ;
iii) cliver l'amorce d'acide nucléique allongée sur le site abasique de manière à générer une extrémité 3'-OH libre qui puisse être allongée par ledit ADN polymérase ; et
iv) répéter les étapes i) à iii) sur les fragments d'ADN ainsi générés.

5. Procédé selon l'une quelconque des revendications précédentes, dans lequel la matrice d'acides nucléiques est de l'ADN.

6. Procédé selon l'une quelconque des revendications précédentes, dans lequel l'amorce d'acide nucléique est une amorce d'ADN.

7. Procédé selon l'une quelconque des revendications 1 à 6, dans lequel les nucléotides précurseurs d'ADN sont sélectionnés parmi le dATP, le dCTP, le dGTP et le dTTP.

8. Procédé selon l'une quelconque des revendications précédentes, dans lequel l'ADN polymérase présente une activité de déplacement de brin.

9. Procédé selon l'une quelconque des revendications précédentes, dans lequel le précurseur d'acide nucléique modifié est le dUTP.

10. Procédé selon l'une quelconque des revendications 4 à 9, dans lequel l'ADN glycosylase est uracile ADN glycosylase (UDG).

11. Procédé selon l'une quelconque des revendications 4 à 10, dans lequel l'acide nucléique allongé est clivé sur le site abasique au moyen d'une enzyme qui clive sur un site abasique d'acide nucléique.

12. Procédé selon la revendication 11, dans lequel l'enzyme est un endonucléase AP.

13. Procédé selon l'une quelconque des revendications précédentes, dans lequel le nucléotide précurseur modifié remplace partiellement l'un des nucléotides précurseurs normaux.

14. Procédé selon l'une quelconque des revendications 1 à 3, 5 à 9 et 13, dans lequel les étapes i) et ii) continuent de manière cyclique jusqu'à ce qu'un des réactifs devienne limitatif.

15. Procédé selon l'une quelconque des revendications 4 à 13, dans lequel les étapes i) à iii) continuent de manière cyclique jusqu'à ce qu'un des réactifs devienne limitatif.

16. Procédé selon l'une quelconque des revendications précédentes, qui est réalisé dans des conditions isothermes.

17. Procédé selon l'une quelconque des revendications précédentes, qui résulte en l'accumulation de fragments monocaténaires en amont déplacés d'acide nucléique spécifiés par les localisations des bases modifiées dans un brin d'acide nucléique complémentaire.

18. Procédé selon l'une quelconque des revendications précédentes pour générer des copies multiples d'amorces monocaténaires discrètes en amont d'une amorce d'acide nucléique initiatrice.

19. Procédé selon les revendications 17 ou 18, dans lequel les fragments déplacés en amont sont allongés dans une réaction secondaire.

20. Procédé selon l'une quelconque de revendications 17 à 19, dans lequel les fragments déplacés en amonts sont allongés sur une matrice d'acides nucléiques secondaire.

21. Procédé selon l'une quelconque des revendications 17 à 20, dans lequel les matrices secondaires multiples sont immobilisées sur une puce à ADN.

22. Procédé selon l'une quelconque des revendications précédentes pour une utilisation en diagnostique de détection.

23. Procédé selon la revendication 22, qui est utilisé pour la détection d'agents pathogènes.

24. Procédé selon la revendication 22, qui est utilisé pour détecter la présence ou l'absence de mutations.

25. Procédé selon la revendication 22, qui est utilisé pour la détection de polymorphismes.

26. Procédé selon l'une quelconque des revendications précédentes pour la quantification du niveau d'un acide nucléique dans un échantillon.

27. Procédé selon l'une quelconque des revendications précédentes pour une utilisation en amplification du signal à partir de tout acide nucléique qui peut fonctionner comme amorce ou matrice.

28. Procédé selon l'une quelconque des revendications précédentes, pour une utilisation en informatique ADN.
